(19) **Europäisches Patentamt / European Patent Office / Office européen des brevets**

(11) **EP 3 773 849 B1**

(12) **EUROPEAN PATENT SPECIFICATION**

(45) Date of publication and mention
of the grant of the patent:
**12.07.2023  Bulletin 2023/28**

(21) Application number: **19776403.8**

(22) Date of filing: **26.03.2019**

(51) International Patent Classification (IPC):
***A61M 16/16*** *(2006.01)*      ***A61M 13/00*** *(2006.01)*

(52) Cooperative Patent Classification (CPC):
(C-Sets available)
**A61M 16/16;** A61M 11/042; A61M 11/044;
A61M 13/003; A61M 16/108; A61M 16/1085;
A61M 16/1095; A61M 2202/0225; A61M 2205/07;
A61M 2205/3334; A61M 2205/3368;
A61M 2205/3633; A61M 2205/502; A61M 2205/75;
A61M 2206/14;                              (Cont.)

(86) International application number:
**PCT/NZ2019/050032**

(87) International publication number:
**WO 2019/190332 (03.10.2019 Gazette 2019/40)**

(54) **HUMIDIFICATION CHAMBER AND APPARATUS**

BEFEUCHTUNGSKAMMER UND VORRICHTUNG

CHAMBRE D'HUMIDIFICATION ET APPAREIL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **26.03.2018   US 201862647966 P**

(43) Date of publication of application:
**17.02.2021   Bulletin 2021/07**

(73) Proprietor: **Fisher & Paykel Healthcare Limited East Tamaki Auckland 2013 (NZ)**

(72) Inventors:
• **SPENCE, Callum James Thomas**
  **Auckland, 2013 (NZ)**
• **VERDOOLD, Vincent**
  **Auckland, 2013 (NZ)**
• **GELL, Zane Paul**
  **Auckland, 2013 (NZ)**
• **BOYES, Richard John**
  **Auckland, 2013 (NZ)**
• **WARNER, Zach Jonathan**
  **Auckland, 2013 (NZ)**

• **BAUMANN, Monika**
  **Auckland, 2013 (NZ)**
• **FISCHER, Christian Francis**
  **Auckland, 2013 (NZ)**
• **IP, Bernard Tsz Lun**
  **Auckland, 2013 (NZ)**
• **MCDERMOTT, Gareth Thomas**
  **Auckland, 2013 (NZ)**
• **DEVA, Karan**
  **Auckland, 2013 (NZ)**
• **GLAVES, Rachael**
  **Auckland, 2013 (NZ)**
• **BARNES, Thomas Heinrich**
  **Auckland, 2013 (NZ)**

(74) Representative: **Treeby, Philip David William et al Maucher Jenkins Seventh Floor Offices Artillery House 11-19 Artillery Row London SW1P 1RT (GB)**

(56) References cited:
**EP-A2- 2 842 589        WO-A1-2007/101298
WO-A1-2007/101298     WO-A1-2017/126982
WO-A1-2017/126982     JP-A- 2004 057 278**

**US-A- 4 943 704      US-A1- 2010 043 791**
**US-A1- 2011 146 216      US-A1- 2016 184 547**
**US-A1- 2017 035 984      US-A1- 2017 035 984**
**US-A1- 2017 197 057      US-A1- 2017 197 057**

(52)  Cooperative Patent Classification (CPC): (Cont.)
A61M 2206/16; A61M 2209/06

C-Sets
A61M 2202/0225, A61M 2202/0007

**Description**

BACKGROUND

1. Field of the Disclosure

The disclosure generally relates to humidification chambers for humidification systems. More specifically, the disclosure relates to humidification chambers for medical use such as in, for example, but not limited to, respiratory and/or surgical humidification systems.

2. Description of Related Art

Many, if not all, existing humidification systems which deliver humidified gases to a patient, or other person in need of such gases, operate under controlled operating parameters. For example, in respiratory systems, it may be desirable to deliver gases to the patient at a particular temperature, pressure, humidity, flow rate, etc.

In surgery, insufflation gases can be used for a variety of purposes. In open surgery, gas can be insufflated into a body cavity for de-airing, such as for example in cardiac surgery. In laparoscopic surgery, the abdominal wall can be distended using gas to provide room for instrument insertion and tissue dissection. During these surgical procedures, it may be desirable to introduce insufflation gas into a surgical cavity under controlled operating parameters such as a particular flow rate, pressure, temperature, or humidity.

Various systems and methods have been developed to humidify and/or warm the gas prior to providing it to the patient. However, these systems and methods have disadvantages which may be addressed by the disclosure.

US 2017/035984 A1 discloses a humidifier includes a humidifier tub with an air inlet and an air outlet. An uppermost end of the air outlet terminates at an exit port positioned in a plane disposed below the air inlet. The air inlet is configured so that air enters the humidifier tub through the air inlet in a lateral direction. The humidifier also includes a base plate provided to a bottom of the humidifier tub. The base plate and humidifier tub define a water chamber adapted to receive a volume of liquid water. The humidifier tub further includes a guidance structure adapted to direct air entering the humidifier tub via the air inlet to swirl downwardly and around within the water chamber and into contact with the liquid water before exiting the exit port. Also acknowledged are US 4943704 A, US 2017/197057 A1, EP 2842589 A2, US 2016/184547, WO 2017/126982 A1, WO 2007/101298 A1, JP 2004057278 A and US 2017/0035984 A1.

SUMMARY

The invention is defined in independent claim 1. Preferred embodiments are disclosed in the dependent claims. It is an object of the disclosure to provide a humidification chamber which at least goes some way towards overcoming the disadvantages of the prior art systems or which will at least provide the public with a useful choice.

According to the present invention there is provided a humidification chamber according to claim 1. Preferred aspects are set out in the dependent claims.

According to a first through sixth aspects of the disclosure there is provided a humidification chamber for use in a medical humidification system, the humidification chamber comprising: a base and a top linked by a side wall to define the chamber; the chamber being configured to contain a volume of water; a gases inlet configured to receive a gases flow from a gases source; and a gases outlet, wherein:

the humidification chamber is configured to increase a path length of gases within the chamber, and/or the residence time of gases within the chamber; and/or
the humidification chamber is configured to increase a velocity of the gases within the chamber, and/or
the humidification chamber is configured to increase a surface area of water available for heat transfer and/or mass transfer to the gases within the chamber, and/or
the humidification chamber is configured to improve a chamber performance over a wide range of flow rates, and/or
the humidification chamber is configured to improve a heat transfer and/or mass transfer to the gases within the chamber, and/or
the humidification chamber and/or the gases inlet is/are configured to introduce the gases flow to the humidification chamber at a direction substantially tangential or adjacent to the side wall of the humidification chamber, such that the gases flow entering the chamber spins around the inside of the chamber.

The gases inlet may be provided in or pass through the sidewall of the chamber.

The gases inlet may be configured to introduce the gases flow into the humidification chamber as a gases jet.

The side wall may define a substantially circular chamber, when viewed from above.

The chamber may comprise an arcuate wall section, the inlet being located adjacent the arcuate wall section, the arcuate wall section being configured to cause the gases introduced into the chamber to travel in an arcuate path, prior to exiting the chamber. The arcuate path may be a circular path when the chamber is viewed from the top.

The gases outlet may be disposed on a top or base/bottom wall of the humidification chamber, preferably at or proximate to the centre thereof. Additionally or alternatively, the gases outlet may be disposed on the side wall of the humidification chamber, optionally facing an opposite way to the direction of the gases flow in the humidification chamber generated by an orientation of the gases inlet and/or flow formations provided in the inlet or the chamber that modify the flow path of the gases.

In accordance with an aspect of this disclosure there is a provided a humidification chamber comprising:

> a base,
> a wall defining a cavity to hold a humidification fluid,
> the wall comprising an arcuate section,
> an inlet located on the wall, the inlet extending in a first direction,
> an outlet located on the wall, the outlet extending in a second direction,
> the second direction being substantially normal/perpendicular to the first direction.

The inlet and outlet may be arranged so as to be orthogonal.

The arcuate wall section may be located between the inlet and the outlet, the inlet being upstream of the arcuate wall section and the outlet being downstream of the arcuate wall section. (Upstream and downstream are in reference to the direction of the gases flow in normal use.) The arcuate section may provide a rotational component to the gases flow. The arcuate wall section may be defined by a cylindrical wall, the gases flow rotating within the chamber prior to exiting the outlet, the inlet and outlet being normal to each other.

The gases inlet comprises a nozzle or is configured to receive a nozzle. According to the invention an inner diameter of the nozzle is configured to decrease along at least a part of a length of the nozzle so as to increase a velocity of the gases flow prior to the gases flow being introduced to the chamber. In other words, the flow path defined by the nozzle may comprise a constriction or restriction. The gases inlet may comprise a substantially tubular body. The constriction may be shaped and configured to reduce resistance to flow. For example the constriction may be internally tapered so as to be gradually narrowed along its length, wherein the internal profile is substantially smooth.

In some examples, the ratio of the inner diameters of the gases outlet to the inner diameter of the gases inlet is between 2:1 to 8:1, and more preferably 3:1 and 7: 1, and in on example is approximately 5:1. The ratio may be selected in dependence upon the type of humidification system.

In some examples the inlet may not decrease in diameter and may be of substantially consistent diameter (or substantially constant cross sectional area) along the length of the inlet. In some examples, the inlet may increase in diameter along the length of the inlet.

In some examples, the gases outlet may have an inner diameter greater than an inner diameter of the gases inlet. A ratio of an inner diameter of the gases inlet to a diameter of a bottom wall of the humidification chamber may be between 1:25 and 1: 10. In another example, the ratio is between 1:20 and 1:15.

The top of the humidification chamber may comprise any one of: a dome shape; a cone shape; an inverted cone shape; and a flat shape, or parts or combinations thereof. The top and/or the gases inlet may comprise an inner surface configured to produce a turbulent gases flow within the humidification chamber. The inner surface of the top and/or sidewall of the humidification chamber, and/or gases inlet may comprise at least one protrusion projecting into the space generally defined by a wall forming the chamber / inlet and/or at least one recess recessed to enlarge the space generally defined by the wall of the chamber / inlet to produce turbulent gases flow in the humidification chamber.

The inlet and side wall may be configured such that gases entering the humidification chamber at the gases inlet swirl into a spiral about an axis within the humidification chamber before exiting the chamber through the gases outlet. Preferably the gases swirl into a spiral about a substantially vertical axis of the humidification chamber. Preferably, the gases swirl into a spiral about an axis that extends from the base to the top of the chamber or vice versa. Preferably, the gases swirl into a spiral about an axis substantially perpendicular to the base of the chamber. For example, the inlet may be disposed on and orientated relative to the side wall such that a distance between adjacent winds / turns of the spiral is reduced when a flow rate of the gases entering the humidification chamber is increased. Alternatively the inlet may be associated with and/or pass through a top of the chamber with flow directors being used to create the desired flow pattern.

The humidification chamber may further comprise at least one insulating layer to prevent or reduce heat loss. In some examples, the at least one insulating layer may comprise a first insulating layer extending over at least part of any of the base, top and/or side wall, optionally the first insulating layer being provided on an external surface of the side wall. In other examples, the humidification chamber may comprise first and second sub-chambers, the second sub-chamber being configured to at least partially surround the first sub-chamber, the first sub-chamber preferably containing the volume of water. Here, the at least one insulating layer may comprise a second insulating layer provided as an air gap formed between the first and the second sub-chambers, although this space may be filled with alternative insulating material as desired.

The flow path through the gases inlet may be angled down towards the base of the humidification chamber so as to increase the volume of gases coming into contact with the water and increase moisture uptake by the gases. Preferably, the gases are not angled directly towards the water (i.e. not vertically) so that the gases are also urged about the inner surface of the chamber side wall, thereby elongating the flow path and/or possibly increasing residence time of gases inside the chamber. Alternatively, the flow path may be inclined upwards towards the top of the chamber on entry of the gases into the chamber. This may create turbulence which promotes mixture of gases inside the chamber and can help in moisture uptake.

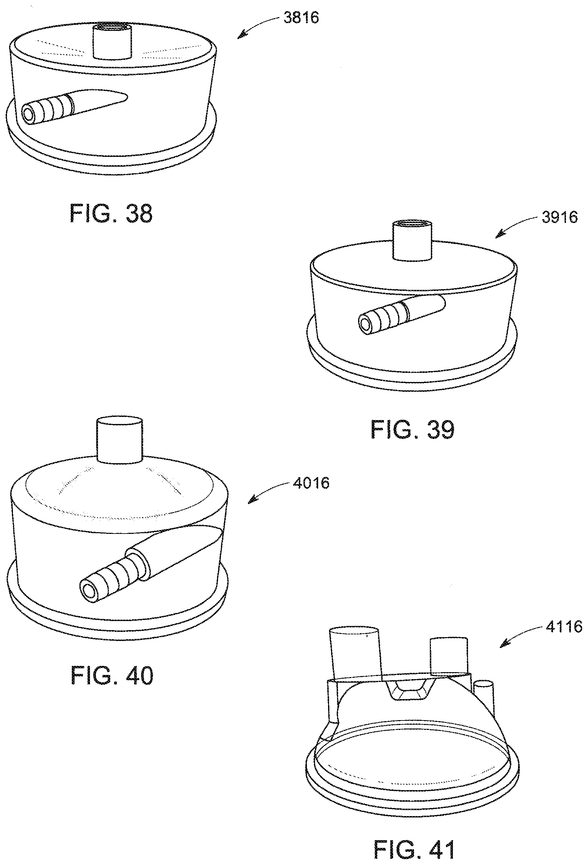

In accordance with aspects of this disclosure, a humidification chamber includes an inlet that "speeds" up the flow and introduces gas flow to the interior of the chamber in a substantially tangential manner such that the flow attaches to the wall and forms a vortex. The vortex flow increases path length of the gases thereby improving humidity. To avoid repetition, references throughout this specification to 'vortex flow' should be taken as including that the path length of the gases within the chamber is increased, as compared to a prior art chamber not configured to provide vortex flow. The increased path length is in comparison to a standard chamber for example an MR225 chamber of Fisher & Paykel Healthcare Limited, or the chamber shown in Fig. 41. For chambers that are of the same volume as each other, the vortex flow creates a longer path length because of the conservation of momentum principle. However, the distance changes due to the velocity differences. In the prior art chamber the velocity of gases is slower as compared to the vortex chamber of the present invention. In the present disclosure the velocity increases thereby causing the distance travelled to increase since the average gases flow i.e. the bulk gases flow takes the same time to come in and out of the chamber.

The humidification chamber may comprise at least one internal element disposed between the gases inlet and the gases outlet to direct or redirect or influence the gases flow within the humidification chamber. In one example, the at least one internal element may be a baffle. In some examples, the at least one internal element may further be configured to heat the gases flow and/or water within the humidification chamber. In some examples, the at least one internal element may be orientated in a plane substantially parallel to the base or the side wall of the humidification chamber.

The base of the humidification chamber may be at least partially formed from or comprise a heat conductive material.

The humidification chamber may comprise a heater plate or a heat conductive plastic casing arranged to at least partially enclose the humidification chamber so as to provide additional heating and/or prevent heat loss.

The humidification chamber may be coupled and/or couplable to a delivery conduit configured to define a gases flow path between a gases source and a patient interface. The humidification chamber may be placed in the gases flow path between the gases source and the patient interface. The delivery conduit may comprise a supply tube defining the gases flow path between the gases source and the humidification chamber and/or a patient conduit defining the gases flow path between the humidification chamber and the patient interface. In some examples, the gases source may comprise a carbon dioxide supply. The patient interface may comprise a trocar or cannula, or a diffuser. In other examples, the gases source may comprise a flow generator. The patient interface may comprise one of: a nasal mask, an oro-nasal mask, an oral mask, a full face mask, a nasal cannula, nasal pillows, and an endotracheal tube.

The humidification chamber may be coupled and/or couplable to and/or associated with a humidification apparatus comprising a base unit comprising a heater plate. The humidification chamber may be removably positioned in contact with the heater plate. In such examples, the base unit may comprise a gases inlet to receive gases from a gases source and a pressurised gases outlet to provide gases to the chamber inlet. The base unit may comprise a humidified gases return port for receiving humidified gases from the chamber, the humidified gases return port being fluidly coupled and/or couplable to an outlet of the base unit that is connectable to a patient interface via a delivery tube, for example. A gases source may integrate with the base unit and/or be couplable thereto, with or without the need for external tubing. Further, the patient interface may connect to the chamber outlet without gases passing through the base unit along their path from the chamber i.e. the delivery tube for the patient interface may connect directly to the chamber outlet.

The gases inlet may comprise one or more walls that define an elongate passageway, wherein the elongate passageway has an inlet end and an outlet end, the inlet end being fluidly couplable to the gases source, including via a conduit, to receive gases therefrom and the outlet end configured to direct gases into the humidification chamber. The elongate passageway may be arcuate along a least a part of its length, preferably at least at or near the outlet end thereof. More particularly, the elongate passageway may have a footprint and/or define a gases path that is parallel to or approximates the contour of at least a portion of the inside of the side wall of the humidification chamber.

The elongate passageway may be tapered along at least a portion of its length, preferably shrinking or reducing in cross-sectional area when moving from a first point to a second point along the passageway, where the second point is nearer the outlet end. Preferably a width and height of the passageway tapers.

At least a part of the elongate passageway may be defined by or coupled to the top of the humidification chamber. In other words, at least part of the passageway may be formed by a top wall of the chamber. Additionally or alternatively, the elongate passageway may be provided to and/or formed integrally with and/or couplable to an opening in the side wall or top of the chamber.

The elongate passageway may at least in part be formed by a cavity that is formed by wall projecting at the top of the chamber. The outlet end may define a circular or elongated opening that faces the base of the humidification chamber and forms the outlet of the passageway. For example, where the passageway is formed by a projection at the top of the chamber, at least a portion of the cavity facing the chamber base may be open. An internal top wall of said walls that define the elongate passageway may be closer to the base of the chamber at a point closer to the outlet end than at the inlet end so as to direct gases towards the chamber base. Additionally or alternatively, a cross-section of an internal top wall of said walls that define the elongate passageway may have a curved or arcuate such that the passageway has a generally concave wall furthest from the chamber base when viewed from inside the chamber.

The gases inlet and outlet may be orthogonal to one another.

The gases inlet may be provided in and/or extend through the side wall of the humidification chamber. Additionally or alternatively, the gases inlet may have at least a portion that defines a smaller cross-sectional area for conveying gases than the gases outlet.

According to a seventh aspect of the disclosure there is provided a humidification chamber for use in a medical humidification system, the humidification chamber comprising: a base and a top linked by a side wall to define the chamber; the chamber being configured to contain a volume of water; a gases inlet, optionally extending through the side wall, configured to receive a gases flow from a gases source; a gases outlet; and one or more elements disposed within the chamber and configured to guide the gases flow along at least a portion of the gas flow path between the gases inlet and the gases outlet of the chamber.

The gases inlet, the gases outlet, and the one or more elements may be arranged such that a length of the gases flow path and/or residence time of the gases within the chamber is increased.

At least one end of the one or more elements may be coupled to one or more of: the base; the top; and the side wall of the chamber.

In some examples, the at least one end may be attached to an inner surface of the side wall and the one or more elements may extend radially from the inner surface of the side wall towards a center of the chamber and/or an opposite side of inner surface of the side wall. The one or more elements may extend in a direction substantially parallel to a water surface. The one or more elements may be disposed above or proximate to a water level and/or to the gases inlet.

In other examples, the at least one end may be attached to an inner surface of the top and/or an inner surface of the base and the one or more elements extend from the inner surface of the top and/or the base towards a center of the chamber and/or to the opposing wall at the base or top of the chamber, respectively. The one or more elements may extend in a direction substantially perpendicular to a water surface.

The gases inlet may be orientated relative to the side wall to introduce the gases flow to the chamber at a direction substantially tangential to the side wall of the chamber, such that the gases flow entering the chamber spins around the chamber over the volume of water.

In some examples, the one or more elements may be disposed within the chamber so as to form a winding or serpentine gases flow path.

In other examples, the one or more elements may comprise one or more openings allowing the gases flow to flow through the one or more elements between the gases inlet and the gases outlet. The one or more openings may be shaped and/or dimensioned and/or disposed on the one or more elements such that a length of the gases flow path within the chamber is increased.

The humidification chamber may further be coupled to a delivery conduit configured to define a gases flow path between a gases source and a patient interface. The humidification chamber may be placed in the gases flow path between the gases source and the patient interface. The delivery conduit may comprise a supply tube defining the gases flow path between the gases source and the humidification chamber and/or a patient conduit defining the gases flow path between the humidification chamber and the patient interface. The delivery conduit may comprise a bubble tube type structure. The delivery conduit may comprise a dual lumen tube with a heater wire in or at the inner lumen. The dual lumen tube may comprise an outer lumen that provides thermal insulation and an inner lumen to transport gases. In some examples, the gases source may comprise a carbon dioxide supply. Other variations as described in relation to the first through sixth aspects are also possible in relation to this aspect. More generally, features of the aspects and optional features thereof may be incorporated in this and subsequent aspects and vice versa.

For surgical applications, the patient interface may comprise a trocar or cannula, or a diffuser. In respiratory assistance or anaesthesia (includes sedation) fields of use, the patient interface may comprise one of: a nasal mask, an oro-nasal mask, an oral mask, a full face mask, a nasal cannula, and nasal pillows. The patient interface may be sealing or non-sealing. For respiratory therapy when the chamber is used in CPAP therapy of BiPAP or other PAP therapy, the interface may comprise a nasal mask, oral mask, full face mask, oro-nasal mask or nasal pillows. The interface for PAP therapy may be an interface that provides a substantial seal with a portion of the user's face. When the chamber is used for respiratory therapy to provide humidified high flow therapy, the interface may be an unsealed interface e.g. a nasal cannula or an oral cannula. The gases source for all embodiments may comprise a flow generator and/or a pressurised gas reservoir and/or a wall gases source, for example of the hospital type comprising a gas outlet in a wall that is connected to a pressurized gases source within the hospital. Both may be used together to provide an additive to ambient air. For example, oxygen may be added to provide oxygen enriched air which is required for some therapies.

The humidification chamber may be coupled and/or couplable and/or associated with a humidifier or base unit comprising a heater plate, as per other aspects / embodiments. The humidification chamber may be removably positioned in contact with the heater plate.

In an eighth aspect of the disclosure there is provided a humidification chamber for use in a medical humidification system, the humidification chamber comprising: a base and a top linked by a side wall to define the chamber; the chamber being configured to contain a volume of water; a gases inlet, optionally extending through the side wall, configured to receive a gases flow from a gases source; a gases outlet; and one or more heating elements disposed within the chamber and/or being coupled to the chamber, wherein the one or more heating elements are configured to increase an overall surface area for heat transfer and/or mass transfer to the gases flow.

The one or more heating elements may be disposed around or below at least a portion of the chamber.

The base and/or at least a portion of the side wall may have a bowl shape and the one or more heating elements may have a corresponding bowl shape.

The base of the chamber may comprise a varying thickness. In some examples, the thickness of a central portion of the base is greater than a thickness of at least a portion of the base adjacent or proximate the side wall.

The one or more heating elements may enclose the chamber.

The one or more elements may comprise a conductive casing configured to be heated by a heat source.

The one or more heating elements may be disposed within the chamber and at least one end of the one or more heating elements may be coupled to a heater plate. In some examples, the heater plate may be provided and/or coupled to the chamber such that the one or more heating elements extend from the base towards a center of the chamber and/or the top of the chamber. The one or more elements may extend in a direction substantially perpendicular to a water surface. The one or more elements may be dimensioned such that at least one portion of the one or more elements is disposed above a water level to heat the gases flow. The one or more elements may comprise a layer having hydrophilic properties such that a thin layer of water covers the at least one portion. The one or more elements may be dimensioned so as to be disposed below a water level. The one or more elements may comprise a linear arrangement of heater fins and/or curved heater fins. A portion of the heating element may extend into the free space of the chamber above the water level. The heating elements may be dimensioned such that they are submerged within the water. The heating elements may be dimensioned such that they extend vertically below a minimum required water level of the chamber.

The gases inlet may be orientated relative to the side wall to introduce the gases flow to the chamber at a direction substantially tangential to the side wall of the chamber, such that the gases flow entering the chamber spins around the chamber over the volume of water.

The humidification chamber may further be coupled to a delivery conduit configured to define a gases flow path between a gases source and a patient interface. The humidification chamber may be placed in the gases flow path between the gases source and the patient interface. The delivery conduit may comprise a supply tube defining the gases flow path between the gases source and the humidification chamber and/or a patient conduit defining the gases flow path between the humidification chamber and the patient interface. The alternative system architectures and forms of patient interface described previously apply equally to this aspect.

The humidification chamber may similarly be coupled and/or coupeable and/or associated with a humidifier or base unit comprising a heater plate. The humidification chamber may be removably positioned in contact with the heater plate.

In a ninth aspect of the disclosure there is provided a humidification chamber comprising:

a base and top linked by a side wall;
an outlet positioned in a central region of either the base or the top wall, wherein the outlet is concentric with the chamber;
an inlet;

wherein the outlet is normal to the inlet.

The outlet may be provided in the base of the chamber. The outlet may extend downwardly from the base of the chamber. The outlet may project into the chamber from the base.

In a tenth aspect of the disclosure there is provided a humidification apparatus for use in a medical humidification system, the humidification apparatus comprising at least two humidification chambers, each of the chambers comprising: a base and a top linked by a side wall to define the chamber; the chamber being configured to contain a volume of water; a gases inlet, optionally extending through the side wall, configured to receive a gases flow from a gases source; and a gases outlet; wherein the gases inlet of at least one but preferably each of the at least two humidification chambers is orientated relative to the side wall to introduce the gases flow at a direction substantially tangential to the side wall, such that the gases flow entering the chamber spins around the chamber over the volume of water.

The gases outlet of a first humidification chamber may be coupled to the gases inlet of a second humidification chamber. The gases inlet of the second humidification chamber may comprise, or may alternatively comprise, the gases outlet of the first humidification chamber. The two chambers may be separate components configured to be removably coupled together, or may be integrally formed with each other or permanently coupled to each other.

The humidification apparatus may further comprise a conduit allowing the gases flow exiting the first humidification chamber at the gases outlet to enter the second humidification chamber at the gases inlet. The conduit may be flexible, or include one or more flexible portions.

The humidification apparatus may further be coupled to a delivery conduit configured to define a gases flow path between a gases source and a patient interface. The delivery conduit may comprise part of a gas delivery or breathing circuit, the breathing circuit comprising the delivery conduit, and a gas supply tube. The humidification apparatus may be placed in the gases flow path between the gases source and the patient interface. The delivery conduit may comprise part of a breathing circuit which also comprises a supply tube defining the gases flow path between the gases source and the humidification apparatus and/or a patient conduit defining the gases flow path between the humidification apparatus and the patient interface. The alternative system architectures and forms of patient interface described previously apply equally to this aspect.

In an eleventh aspect of the disclosure there is provided a humidification chamber for use in a medical humidification system, the humidification chamber comprising: first and second internal or sub-chambers, each of the chambers comprising a base and a top linked by a side wall to define the respective internal or sub-chamber; at least one of the first and second internal or sub-chambers being configured to contain a volume of water; at least one of the first and second internal or sub-chambers comprising a gases inlet, optionally extending through the side wall, configured to receive a gases flow from a gases source; and at least one of the first and second internal or sub-chambers comprising a gases outlet configured to allow the gases flow to exit the humidification chamber; wherein the gases inlet is orientated relative to the side wall to introduce the gases flow to the humidification chamber at a direction substantially tangential to the side wall of the humidification chamber, such that the gases flow entering the humidification chamber spins around the humidification chamber over the volume of water.

One or more internal elements may be disposed within the first internal chamber that are configured to guide the gases flow along at least a portion of the gases flow path between the inlet and the outlet of the chamber. The one or more internal elements may comprise one or more heater elements configured to heat the gases flow flowing through the first internal chamber. The one or more internal elements may be disposed within the first internal or sub-chamber such that a length of the gases flow path within the humidification chamber is increased.

The at least one end of the one or more internal elements may be coupled to one or more of: the base; the top; and the side wall of the chamber. In some examples, the at least one end may be attached to an inner surface of the top and/or an inner surface of the base and the one or more internal elements extend from the inner surface of the top and/or the base towards a center of the first internal chamber and/or beyond the centre towards the opposing wall formed by the other of the base and top, respectively.

The one or more internal elements may be disposed within the chamber so as to form a winding or serpentine gases flow path.

The one or more internal elements may comprise one or more openings allowing the gases flow to flow through the one or more elements between the gases inlet and the gases outlet. The one or more openings may be shaped and/or dimensioned and/or disposed on the one or more internal elements such that a length of the gases flow path within the humidification chamber is increased.

The first internal or sub-chamber may comprise: a first gases inlet configured to receive the gases flow from the gases source; and a first gases outlet configured to allow the gases flow to exit the humidification chamber. The second internal or sub-chamber may comprise: a second gases inlet configured to receive the gases flow from the first internal chamber; and a second gases outlet configured to allow the gases flow to exit the second internal or sub-chamber. The first internal or sub-chamber may at least partially or completely enclose the second internal or sub-chamber. In one example, at least a portion of the gases flow may flow around the outer surface of the side wall and/or top of the second internal or sub-chamber before exiting the humidification chamber at the first gases outlet. In another example, the at least a portion of the gases flow may enter the second internal or sub-chamber at the second gases inlet prior to exiting the second internal or sub-chamber at the second gases outlet.

The second internal or sub-chamber may comprise the gases inlet configured to receive the gases flow from the gases source and the gases outlet configured to allow the gases flow to exit the humidification chamber. The first internal or sub-chamber may at least partly, or completely, surround the second internal or sub-chamber so as to prevent heat loss in the second internal or sub-chamber. The second internal or sub-chamber may comprise one or more heater elements. The one or more heater elements may be disposed on a wall separating the first and second internal or sub-chambers.

The humidification chamber may further be coupled to a delivery conduit configured to define a gases flow path between a gases source and a patient interface. The humidification chamber may be placed in the gases flow path between the gases source and the patient interface. The delivery conduit may comprise a supply tube defining the gases flow path between the gases source and the humidification chamber and/or a patient conduit defining the gases flow path between the humidification chamber and the patient interface. Other system features and architectures, including forms of patient interface may be taken from the earlier aspects. The same applies to coupling and/or association of the chamber with a humidifier or base unit comprising a heater plate, wherein the humidification chamber is preferably removably positionable in contact with the heater plate.

In a twelfth aspect of this disclosure there is provided a humidification chamber for use in a medical humidification system, the humidification chamber comprising:

a base and a top linked by a side wall to define the chamber;

a gases inlet configured to receive a gases flow from a gases source; and
a gases outlet,
wherein the gases inlet has a longitudinal axis which is substantially parallel to the tangent to the side wall and is located adjacent the side wall;

wherein the gases inlet is configured to introduce the gases flow to the humidification chamber adjacent the side wall at a velocity sufficient to causes the gases to attach to the side wall.

In a thirteenth aspect of this disclosure there is provided a humidification chamber for use in a medical humidification system, the humidification chamber comprising:

a base and a top linked by a side wall to define the chamber;
a gases inlet configured to receive a gases flow from a gases source; and
a gases outlet,
wherein the gases inlet is configured to introduce the gases flow to the humidification chamber at a direction non-orthogonal to the side wall of the humidification chamber, such that a flow path length of the gases flow through the chamber between the gases inlet and gases outlet, is increased.

In a fourteenth aspect of the disclosure there is provided a medical insufflation system comprising a humidification apparatus, the humidification apparatus comprising a humidification chamber as defined in any one of the preceding aspects and examples.

The medical insufflation system may further comprise any one or more of: a gases source; a patient interface; and a delivery conduit configured to define a gases flow path between the gases source and the patient interface.

The humidification apparatus may be configured to be placed in the gases flow path between the gases source and the patient interface.

The delivery conduit may comprise a supply tube defining the gases flow path between the gases source and the humidification apparatus and/or a patient conduit may define the gases flow path between the humidification apparatus and the patient interface.

The medical insufflation system may further comprise a connector provided between the delivery conduit and the patient interface. The connector may be a Luer lock connector comprising at least one end configured to be coupled to a patient interface fitting, the at least one end being further configured to lock and seal around an outer surface of the patient interface fitting when the Luer lock connector is coupled to the patient interface. The at least one end may form a seal with the outer surface of the patient interface fitting when the at least one end and the patient interface fitting are coupled.

The medical insufflation system may further comprise a filter assembly forming part of the gases flow path, the filter assembly comprising: a filter medium operative to filter medical gases; a housing comprising an inlet, an outlet and the filter medium, the housing defining a gases flow path through the filter medium between the inlet and the outlet; and at least one heating element being positioned in the housing and being configured to heat the filter medium; wherein, the at least one heating element is spaced apart from the filter medium and from an inner surface of the housing.

In some examples, the patient interface may comprise a trocar or a cannula for laparoscopic surgery. In other examples, the patient interface may comprise a diffuser for use in open surgery.

The gases source may comprise a carbon dioxide supply.

The humidification chamber may be coupled and/or associated with a humidifier or base unit comprising a heater plate. The humidification chamber may be removably positioned in contact with the heater plate.

In a fifteenth aspect of the disclosure there is provided a kit of parts for an unassembled medical insufflation system, the kit comprising a humidification chamber as defined in any one of first ten aspects and examples, and any one or more of: a breathing circuit comprising one or more conduits configured to define a gases flow path between a gases source and a patient interface; the breathing circuit comprising a gas delivery conduit and a supply conduit defining a gases flow path between the gases source and a humidification apparatus comprising the humidification chamber. The kit of parts may further comprise an expiratory circuit comprising an expiratory conduit configured to delivery gases from the patient to the gas source, to another device, or to the ambient environment. The or at least part of, the expiratory conduit may be breathable.

The kit of parts may further comprise a filter assembly (which may further comprise a delivery tube connector at a gases source end of the delivery tube and/or a delivery tube connector at a patient interface end of the delivery tube), a connector configured to couple the delivery conduit and the patient interface, a humidifier or base unit comprising a heater (the heater optionally being in the form of a heater plate, the humidification chamber being removably positionable in thermal contact with the heater) and a gases source (e.g. a flow generator).

In a sixteenth aspect of the disclosure there is provided a respiratory gases delivery system comprising a humidification apparatus, the humidification apparatus comprising a humidification chamber as defined in any one of the first through ten aspects.

The respiratory gases delivery system may further comprise a flow generator being in fluid communication with the humidification chamber, the flow generator being configured to generate the gases flow. The flow generator may be controlled to provide a desired flow. The flow generator may comprise a flow controlled gas source that controls to a set flow. Alternatively, or additionally, the gases source may be controlled to provide a desired pressure or a set pressure.

The humidification chamber may be configured to humidify the gases flow prior to delivery to a patient.

The humidification chamber may be located downstream of the flow generator.

The respiratory gases delivery system may further comprise: a patient interface; and a breathing circuit configured to define a gases flow path between the flow generator and the patient interface. The breathing circuit may comprises a supply tube defining the gases flow path between the flow generator and the humidification apparatus and/or a patient conduit defining the gases flow path between the humidification apparatus and the patient interface.

The patient interface may comprise one of: a nasal mask; an oro-nasal mask; an oral mask; a full face mask; a nasal cannula; and nasal pillows.

The humidification chamber may be coupled and/or associated with a humidifier or base unit comprising a heater plate. The humidification chamber may be removably positioned in contact with the heater plate.

In a seventeenth aspect of the disclosure there is provided a humidification system comprising: a humidifier or base unit comprising a heater plate; and a humidification chamber as defined in any one of first ten aspects and examples. The humidification chamber may be removably positioned in contact with the heater plate.

The humidification system may comprise any one or more of:

a respiratory humidification system;
an anesthesia humification system configured to provide humidification during anesthesia;
a CPAP humidification system;
a high flow therapy humidification system;
a surgical humidification system;
a medical humidification system, for example for use during other medical procedures such as upper GI procedures or endoscopy.

Any of the humidification chambers of the first to thirteenth aspects, may be configured to be used with, or comprise part of, any one or more of the systems set out above.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

To those skilled in the art to which the disclosure relates, many changes in construction and widely differing embodiments and applications of the disclosure will suggest themselves without departing from the scope of the disclosure as defined in the appended claims. The disclosures and the descriptions herein are purely illustrative and are not intended to be in any sense limiting.

BRIEF DESCRIPTION OF THE DRAWINGS

Preferred forms of the disclosure will now be described with reference to the accompanying drawings in which:

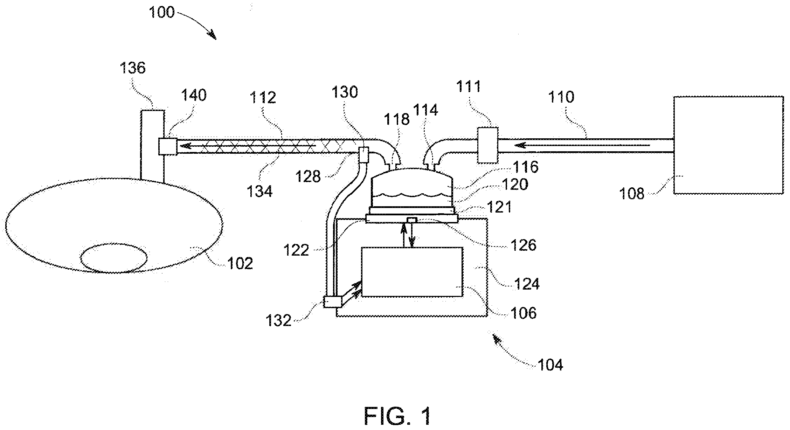

Figure 1 is a schematic view of an embodiment of an insufflation system comprising a humidifier and/or a humidification chamber;

Figure 1a is a schematic view of an embodiment of an anesthesia system comprising a humidifier and/or a humidification chamber;

Figure 1b is a schematic view of an embodiment of a PAP respiratory gases system comprising a humidifier and/or a humidification chamber;

Figure 1b is a schematic view of an embodiment of a high flow respiratory gases system comprising a humidifier and/or a humidification chamber;

Figures 2 to 8 are schematic views of embodiments of a humidification chamber;

Figure 9 is a schematic partly cross-sectional view another embodiment of a humidification chamber;

Figure 10A is a schematic cross-sectional view of a further embodiment of a humidification chamber;

Figure 10B is an isometric view of the humidification chamber of Fig. 10A;

Figure 11 is a schematic side view of another embodiment of a humidification chamber;

Figures 12 and 13 are schematic partly cross-sectional views of other embodiments of humidification chambers;

Figure 14A is a schematic side cross-sectional view of a further embodiment of a humidification chamber;

Figure 14B is a schematic top cross-sectional view of the humidification chamber of Fig. 14A;

Figure 15A is a schematic side cross-sectional view of another embodiment of a humidification chamber;

Figure 15B is a schematic top cross-sectional view of the humidification chamber of Fig. 15A;

Figures 16 to 19 are schematic top cross-sectional views of other embodiments of humidification chambers;

Figures 20A and 20B are schematic cross-sectional views of another embodiment of a humidification chamber;

Figure 21 is a schematic partly cross-sectional view of another embodiment of a humidification chamber;

Figures 22 to 24 are schematic cross-sectional views of other embodiments of humidification chambers;

Figure 25 is a schematic top cross-sectional view of another embodiment of a humidification chamber;

Figures 26A to 30 are schematic cross-sectional views of other embodiments of humidification chambers;

Figure 31 is a schematic partly cross-sectional view of another embodiment of a humidification chamber;

Figures 32 to 35 are schematic views of inlet arrangements of other embodiments;

Figure 36 is a graph illustrating the efficiency at different flow rates of embodiments of humidification chambers, constructed and operative in accordance with the disclosure;

Figures 37 to 40 are side views of other embodiments of humidification chambers; and

Figure 41 is a side view of a prior art humidification chamber used in the Fisher & Paykel SH870 surgical humidification system.

Figures 42 to 44 are perspective, top and bottom (with the base removed) views of a humidification chamber according to a further embodiment.

Figure 45 is a side view of a modified form of the humidification chamber of Figs. 42 to 44 in accordance with a further embodiment.

Figures 46 to 48 provide perspective, top and bottom views of a humidification chamber according to another embodiment, with the chamber base plate removed to show internal detail.

Figure 49 shows a portion of a chamber according to another embodiment.

Figure 50 shows an inlet of a humidification chamber according to another embodiment.

Figure 51 is a graph showing test results of absolute humidity vs flow rate, for the MR225 chamber of Fisher & Paykel Healthcare Limited, and a humidification chamber according to the current disclosure.

Figure 52 is a graph showing test results of relative humidity vs flow rate, for the MR225 chamber of Fisher & Paykel Healthcare Limited, and a humidification chamber according to the current disclosure.

Figure 53 is a graph showing test results of dewpoint vs flow rate, for the MR225 chamber of Fisher & Paykel Healthcare Limited, and a humidification chamber according to the current disclosure.

Figure 54 is a graph showing test results of gas temperature vs flow rate, for the MR225 chamber of Fisher & Paykel Healthcare Limited, and a humidification chamber according to the current disclosure.

Figure 55 is a schematic cross sectional view of another humidifier chamber according to an embodiment of this disclosure.

Figure 56 is a schematic view of an anesthesia humidification system according to the current disclosure.

Figure 57 is a schematic view of a high flow respiratory therapy humidification system according to the current disclosure.

Figure 58 is a schematic view of respiratory therapy humidification system according to the current disclosure.

DETAILED DESCRIPTION

In the following description, numerous specific details are set forth in order to provide a thorough understanding of the various principles of the disclosure. However, those skilled in the art will appreciate that not all these details are necessarily always required for practicing embodiments according to the disclosure.

Although the principles disclosed are largely described herein in relation to laparoscopy or open surgery procedures, this is an example selected for convenience of presentation, and is not limiting. The humidifiers and/or humidification chambers described herein may be used for any suitable medical/surgical procedure and in any suitable medical humidification system comprising a gas delivery circuit, such as for example invasive ventilation, non-invasive ventilation, high flow delivery, positive pressure delivery (e.g. CPAP).

Reference is now made to Fig. 1, which is a schematic view of a medical insufflation humidification system including a humidifier and/or a humidification chamber constructed and operative in accordance with an embodiment.

Fig. 1 illustrates an insufflation humidification system 100 for delivering a temperature- and humidity-controlled gases flow to a patient 102, the insufflation system 100 having a humidification apparatus or humidifier 104 incorporating a humidifier control system 106. The humidifier 104 is connected to a gas source 108 through an inlet conduit 110. The humidifier 104 delivers humidified gas to the patient 102 through a patient or gas delivery conduit 112. The conduits 110, 112 can be made of flexible plastic tubing. The conduits 110, 112 comprise part of a breathing circuit being a gas flow path for delivering gas from the gas source 108 to the patient.

The gas source 108 could be integral to the system 100 and comprise a flow generator including a blower for example (i.e. the gas source 108 and the humidifier 104 may be combined to form a single apparatus or the two separate components may be modular and configured to couple together without the need for separate conduit(s) therebetween, as is known in the art). Alternatively, or additionally the gas source could be separate but connected to the system 100 and comprises a hospital compressed gas source for example.

The humidifier 104 can receive gas from the gas source 108 through the gas supply or inlet conduit 110. The gas can be filtered through a filter 111 and delivered to the humidifier 104 through a humidifier inlet 114. The gas is humidified as it passes through a humidifying chamber 116, which is effectively a water bath, and the gas flows out through a humidifier outlet 118 and into the patient conduit 112. The gas then moves through the patient conduit 112 to the patient 102 via a connector 140 (e.g. Luer connector) and the patient interface 136. The patient interface 136 may be, for example, but not limited to, a trocar or cannula for laparoscopic surgery or a diffuser for open surgery. Patient conduit 112 comprises a gas delivery conduit comprising part of a breathing circuit comprising one or more conduits or tubes forming a gas flow path between the flow generator and the patient, including gas supply conduit 110.

The humidifier chamber 116 may be removably engageable with a body 124 of the humidifier 104. The humidification chamber 116 may comprise a side wall, and a base/bottom. The humidification chamber 116 may further comprise a top, an openable lid, or may be topless (e.g. open topped, in which case, the humidification chamber 116 may be received in a sealable cavity of the humidifier body 124). The top, base and side walls may together define a substantially circular chamber when the chamber is viewed from the top. The side wall is arcuate in shape and may define other arcuate shapes. For example, the chamber may be an elliptical chamber when viewed from the top, or any other chamber having an arcuate but not necessarily perfectly circular shape when viewed from above. The chamber may be symmetrical about at least one axis when viewed from the top. Preferably the chamber is symmetrical about 2 axes, that is, a horizontal and a vertical axis when viewed from above.

The base 121 of the humidification chamber 116 may have a heat conductive (e.g. metal) region or may be entirely heat conductive, and may be adapted to hold a volume or reservoir of water 120, which can be heated by a heater plate 122. The heater plate 122 may be in thermal contact with the heat conductive base 121 of the humidification chamber 116. Providing power to the heater plate 122 can cause heat to flow from the heater plate 122 to the water 120 through the heat conductive base 121. As the water 120 within the humidification chamber 116 is heated it can evaporate and the evaporated water can mix with gases flowing through the humidification chamber 116 from the gas source 108, optionally via the filter 111. Accordingly, the humidified gases leave the humidification chamber 116 via outlet 118 and are passed to the patient 102 via the patient conduit 112, the connector 140, the patient interface 136 and into the surgical site to, for example, insufflate the surgical site and/or expand a body cavity.

The humidifier 104 includes a humidifier control system 106 configured to control a temperature and/or humidity of the gases being delivered to the patient 102. The humidifier control system 106 can be configured to regulate an amount of humidity supplied to the gases by controlling an electrical power supplied to the heater plate 122. The humidifier control system 106 can control operation of the humidification system 104 in accordance with instructions set in software and in response to system inputs. System inputs can include a heater plate sensor 126, a chamber outlet temperature sensor 128, and a chamber outlet flow sensor 130. For example, the humidifier control system 106 can receive temperature information from the heater plate sensor 126 which it can use as an input to a control module used to control the power or temperature set point of the heater plate 122. The humidifier control system 106 can be provided with inputs of temperature and/or flow rates of the gases. For example, the chamber outlet temperature sensor 128 can be provided to indicate to the humidifier control system 106 the temperature of the humidified gases as they leave the outlet 118 of the humidification chamber 116. The temperature of the gases exiting the chamber can be measured using any suitable temperature sensor 128, such as a wire-based temperature sensor. The chamber outlet flow sensor 130 can be provided to indicate to the humidifier control system 106 the flow rate of the humidified gases. The flow rate of the gases through the chamber 116 can be measured using any suitable flow sensor 130, such as a hot wire anemometer, or a thermistor configured for use as a flow sensor. In some embodiments, the temperature sensor 128 and flow sensor 130 are in or otherwise provided to the same sensor housing. The temperature sensor 128 and flow sensor 130 can be connected to the humidifier 104 via connector 132. Additional sensors may be incorporated into the insufflation system 100, for example, for sensing parameters at the patient end of the patient conduit 112. Alternatively the sensors may be wirelessly coupled to the controller. Alternatively the connector may be internal, that is, the sensors are coupled to the controller by integrated wires rather than an external cable.

The humidifier control system 106 can be in communication with the heater plate 122 such that the humidifier control system 106 can control a power delivered to the heater plate 122 and/or control a temperature set point of the heater plate 122. As described further herein, the humidifier control system 106 can determine an amount of power to deliver to the heater plate 122, or a heater plate set point, based at least in part on any one or more of a flow condition, an operation mode, a flow reading, an outlet temperature reading, a heater plate sensor reading.

The insufflation system 100 can include a conduit heating wire 134 configured to provide heat to the gases traveling along the patient or gas delivery conduit 112. Gases leaving the outlet 118 of the humidification chamber 116 can have a high relative humidity (e.g., about 100%). As the gases travel along the patient conduit 112 there is a chance that water vapor may condense on the conduit wall, reducing the water content of the gases. To reduce condensation of the gases within the conduit, the conduit heating wire 134 can be provided within, throughout, and/or around the patient conduit 112, including being at least partly within a wall forming the patient conduit 112. Power can be supplied to the conduit heating wire 134 from the humidifier 104 and can be controlled through the humidifier control system 106. In some embodiments, the heating wire 134 is configured to maintain the temperature of the gas flowing through the patient conduit 112. In some embodiments, the conduit heating wire 134 can be configured to provide additional heating of the gases to elevate the gases temperature to maintain the humidity generated by the heated water bath in the humidifier 104.

The gas delivery conduit may be single walled or comprise a dual conduit configuration that includes an outer conduit and an inner conduit. The inner conduit may carry the gases and include the heating wire 134. The outer conduit may provide insulation of the inner conduit. The outer conduit and inner conduit may be co-axial and there may be an air gap between the outer conduit and the inner conduit.

A further filter or filter assembly (not shown) may be optionally provided and disposed in use between the humidifier 104 and the patient interface 136 so as to allow re-use of the inlet conduit 110 and/or an inspiratory conduit, and in some instances re-use of the humidification chamber 116. The filter assembly can comprise a filter medium for filtering the gases exiting the outlet 118 of the humidification chamber 116. The filter assembly may also comprise a housing and a heating element. The filter medium can be positioned inside the housing so that the humidified gases flowing through the housing are filtered and particles removed. The heating element can be positioned in the gases flow path between the inlet and the outlet of the housing but spaced apart from the filter medium and the housing. The heating element can be configured to heat the filter medium to reduce condensation and prevent the filter becoming clogged. Such filters are described in, for example, WO2018/106127.

The connector 140 can be a Luer connector comprising a body having an interior region defining a gases flow passageway allowing insufflation gases to flow through. The body can comprise a first end that removably connects to a fitting of the patient interface 136 and a second end that attaches, preferably permanently, to the tubing of the patient conduit 112. It will be appreciated that the Luer connector 140 of Fig. 1 can be a high flow Luer connector providing particular sealing and retention features with less resistance to gases flow than traditional Luer connectors of the art. Embodiments of such high flow Luer connectors are described, for example, in WO2018097738. In some embodiments, the humidifier chamber 116 of the humidifier 104 can be configured to provide a controlled gases flow pattern for the insufflation gases. The humidification chamber 116 can be configured to cause the gases entering the humidification chamber 116 via the inlet 114 to swirl in a vortex (i.e. spiral) within the chamber 116 as they exit the chamber 116 via the outlet 118. For example, the inlet 114 may be provided in the side wall and orientated relative to the side wall such that the gases flow enters the humidification chamber 116 via the inlet 114 at a direction substantially tangential to the side wall. In other words, the inlet 114 may be positioned on the humidification chamber 116 and orientated relative to the side wall such that the gases flow along the internal surface of the side wall when entering the humidification chamber 116. Having the gases swirling in a vortex i.e. spiral within the humidification chamber 116 improves the performance of the humidifier 104, and by extension of the insufflation system 100, by increasing the efficiency of the humidification chamber 116. In some embodiments, the inlet 114 may be provided in the side wall of the chamber 116, with the outlet 118 distal from the inlet 114, with the inlet 114 configured such that the direction of gases flow as that flow exits the inlet 114 and enters the chamber 116 is not aligned with the side wall. The inlet 114 is arranged such that the gases are introduced adjacent a tangential to a side wall such that the gases introduced into the chamber attach to the wall of the chamber and travel around the chamber to create a spiral flow. In other embodiments, the inlet 114 may be provided fully in the side wall of the chamber 116.

Additionally, and/or alternatively, the inlet 114 may be angled (e.g. angled down towards the bottom of the humidification chamber 116) such that the gases entering the humidification chamber 116 at a direction substantially tangential to the side wall are further pushed towards the water 120.

Lastly, in some embodiments, the vertical position of the inlet 114 in the side wall may be varied so as to provide different gases flow patterns for the insufflation gases within the humidification chamber 116.

In one embodiment, the top of the humidification chamber 116 comprises a dome shape configured to reflect any upwards moving gases back into the chamber 116. This dome shape may increase the length of the gases flow path within the chamber 116 and may make it harder for the gases to exit the chamber 116. The dome shape of the top of the chamber 116 may increase the length of the gases flow path of the bulk gases flow since the gases are reflected into the chamber 116 and cause the gases to move around the chamber 116 for longer, thereby increasing the path length. Additionally, and/or alternatively, the inlet 114 can be configured to provide an increased velocity to the gases entering the humidification chamber 116. For example, but not limited to, the inlet 114 can be dimensioned so as to accelerate the velocity of the gases passing through it. In another example, the inlet 114 can be configured to introduce the gases into the humidification chamber 116 as a gases jet. Additionally and/or alternatively the inlet may be nozzle shaped, or have a nozzle shaped portion wherein the internal surface of the inlet tapers or inclines such that the cross sectional area of the inlet decreases along its length. Additionally, and/or alternatively, the inlet 114 can be provided in a side wall of the humidification chamber 116 such that the direction of the gases flow entering the chamber 116 is substantially tangential to the side wall and the outlet can be positioned on the upper wall (e.g. at an apex of the dome-shaped upper wall). This configuration enables the gases entering via the inlet 114 to swirl in a vortex i.e. spiral within the humidification chamber 116 before exiting by the outlet 118.

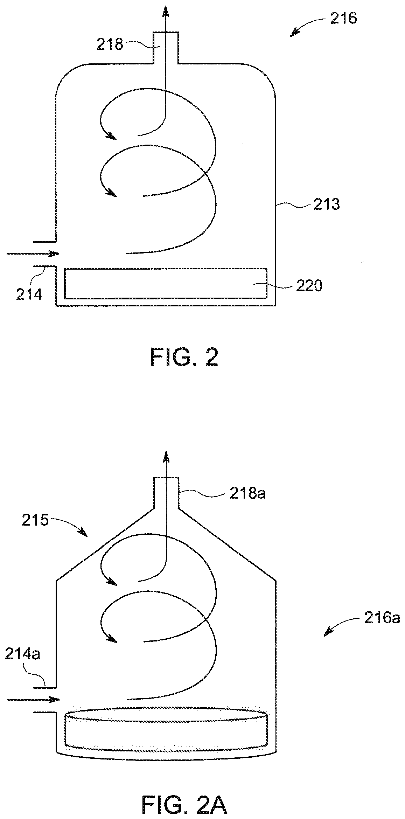

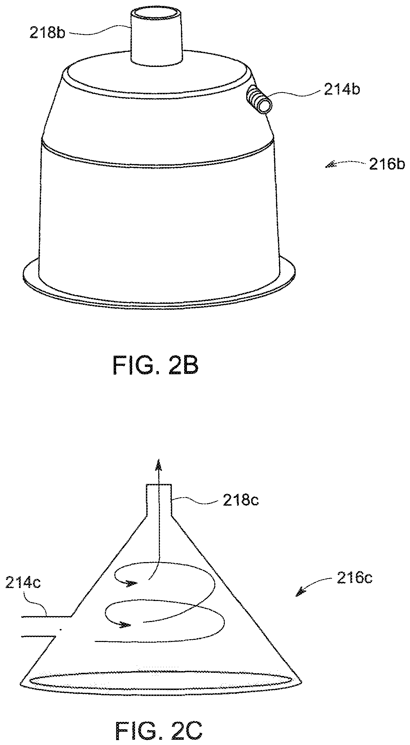

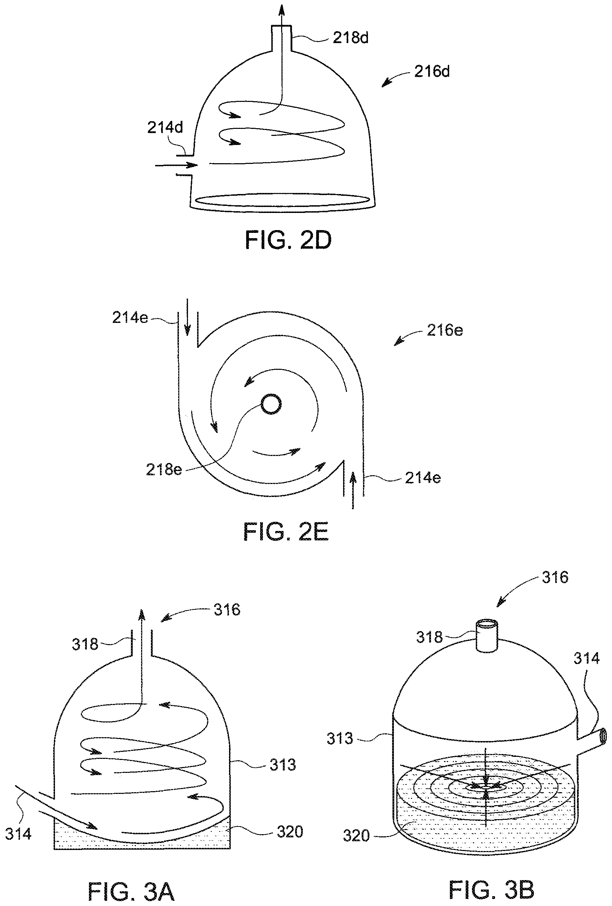

Figs. 2 to 35 illustrate a number of embodiments of the humidification chamber 116 and/or humidifier 104, that improve the efficiency of the humidification chamber 116 by maximizing the residence (dwell) time, and/or increasing the gases velocity, and/or increasing the surface area available for heat transfer and/or mass transfer, and/or improving the performance over a wide range of flow rates, and/or improving the heat transfer and/or mass transfer, and/or increasing the gas flow path in the chamber. Movement of the humidification fluid within the chamber improves heating of the humidification fluid, the movement of the fluid promoting mixing of the fluid, improving heat transfer and/or mass transfer and improving vaporization. The chamber may increase efficiency of vaporization of the fluid and may increase the rate of vaporization for a given power input to the heater plate. The water in the chamber moves in a vortex type manner. This causes convection within the water and moves water relative to the stationary conductive base of the chamber, thereby improving heat transfer and/or mass transfer to the surface of the water. This also improves the mass transfer from the water to the gases flow. This improves creation of water vapour (i.e. vaporization) and thereby improves humidification, as well as to shorten and/or reduce the warm up period of the chamber to reach a steady state.

Maximizing the residence or dwell time and/or flow path length can be achieved by positioning the inlet and outlet on the chamber such that a vortex flow is created within the humidification chamber. The vortex allows for an extensive residence time, thereby increasing moisture pick-up. The humidification chamber may therefore increase the residence time of the gases in the chamber, The humidification chamber may maximise the path length of the gas flow within the chamber, and may therefore provide one, some or all, of the advantages stated herein. This can be achieved by positioning the inlet and outlet on the chamber such that a vortex flow is created within the humidification chamber. The vortex nature of the gases flow in the chamber i.e. the rotating/spinning flow for repeated rotations of gas within the chamber increases the distance travelled by the gas within the chamber, before the gas flows through the chamber outlet. The gas is therefore in contact with the wetted surfaces over a greater distance, increasing the opportunity for humidification. The increased contact with the wetted surface area can lead to more efficient humidification of the gases.

Conservation of mass in the sealed chamber requires that what goes in must equal to what goes out. The vortex flow may also increase the residence time of the gases in the chamber thereby increasing the moisture pick up. However, the vortex may encourage the humidified gases to exit the humidification chamber before the non-humidified gases. Furthermore, warm gases are less dense (i.e. lighter) than cool gases. When the gases are rotating, the centripetal acceleration causes a centripetal force on the gases. This centripetal force ($F_C$) is proportional to the gases density ($\rho$), velocity (v) and radius of curvature (r) and is given by the following equation:

$$F_C = \frac{\rho v^2}{r}$$

Therefore, the cool and yet not humidified gases experience a greater centripetal force, driving them towards the outside of the chamber and away from the central exit outlet. Conversely, the warmed and humidified gases experience less centripetal force and exit preferentially from the central exit outlet.

The gases velocity can be maintained by providing the inlet at a position on the side wall of the humidification chamber such that the direction of the gases flow is substantially tangential to the side wall and the outlet at a central location on the upper wall. In addition, increasing the gas flow path over a liquid within the chamber also increases the moisture pick-up. The entire water surface area is therefore exposed to fast moving gases which increases the humidification efficiency. Increasing the gases velocity increases the Reynolds number and turbulence of the gases flow in the chamber. Increasing the turbulence increases mixing and disrupt the boundary layer between the liquid and gases, thereby reducing the relative humidity and increasing the humidity gradient close to the water surface. Lastly, increasing the gases velocity may cause the distance between adjacent winds of the spiral/vortex to reduce, therefore increasing the residence time and/or gas flow path. Adjacent winds could include stacked winds on top and below one another along the central longitudinal axis of the chamber; or concentric winds adjacent one another along a single plane.

The vortex or spiraling flow increases moisture pick-up via surface area by increasing the efficient use of the surface area available for heat transfer and/or mass transfer and/or increasing the actual surface available for heat transfer and/or mass transfer. Increasing the actual surface area can be achieved by causing ripples in the water surface and, at high flow rates, disrupting the liquid surface tension to cause the water to splash. The vortex causes the entirety of, or more of, the gases within the chamber to circulate and there are at least less regions of stagnant gases flow. Therefore, the entire surface area of the liquid is being exposed to gases moving over it and picking-up moisture.

Improving the heat transfer and/or mass transfer can be achieved by the movement of water. The rotation of the gases induces a rotation of the water due to viscous shear. The movement of the water over the chamber improves the efficiency of the heat transfer and/or mass transfer from the heater plate into the water itself. This is advantageous because a lower heater plate temperature will be necessary for a desired heat input, or heat transfer and/or mass transfer may improved for a given heat input. Additionally or alternatively, the movement of water causes its mixing which homogenizes the heat distribution in and throughout the water, and/or homogenizes the water faster compared to a non-vortex chamber, e.g. the SH870 chamber shown in Fig. 41. This is due to shear and turbulent mixing induced in the water as part of its rotation (includes but not limited to rotation about its horizontal and/or vertical axis) driven by the flow of gases through the chamber.

Increasing the gas flow path within the chamber improves moisture pick-up from the water in the chamber. The gases flow path is increased as the gases travel in a circular or rotational direction in a spiral/vortex, from a region proximate to the side wall of the chamber to the outlet, which may be located at a centre or central region of the chamber. The increased gas flow path increases the time and/or distance the gases flow is in contact with the water, thereby improving the efficiency of moisture pick-up.

Further, improving the performance over a wide range of flow rates can be achieved by using a vortex gases flow within the humidification chamber. Humidification chambers typically suffer performance issues at high flow rates as these tend to reduce path length and/or the residence time of gases inside the humidification chamber. The vortex humidification chamber provides a more reliable performance across a wide range of flow rates as will be explained hereinafter. The vortex chamber may also be advantageous because the vortex flow provides for improved humidification of the gases at low flow rates, that is, when using a respiratory gases delivery system for neonates or infants. The vortex chamber can improve humidification by making humidification faster at low flows or increase the humidity imparted to the gases. Prior art chambers also have a relatively large variance in residence time from an average residence time because some gas particles travel straight from the inlet to the outlet while other remain stagnant in the chamber. The vortex chamber of the present disclosure can reduce the variance in the residence time, because most of the gases are spinning in the chamber. This reduces stagnant regions and causes most of the gas to reside in the chamber for approximately the average residence time. The lower variance is advantageous because most of, or more of, the gases move along the longer path length and are exposed to the wet surface for a similar time leading to improved humidification. The present disclosure provides a more consistent residence time for the bulk gases flow.

Reference is now made to Figs. 2 to 19, which are examples of embodiments of humidification chambers. Whilst these embodiments and features have been described separately, it is within the scope of this disclosure to combine two or more features/embodiments together. The humidification chambers depicted in Figs. 2 to 19 have features configured to increase the efficiency of a humidification chamber by increasing the residence or dwell time and/or otherwise increasing moisture uptake and/or heat transfer and/or mass transfer and/or increasing the length of the gases flow path.

Figs. 2 to 5 illustrate different arrangements for the inlet and outlet ports of a humidification chamber. In one embodiment depicted in Fig. 2, the inlet 214 is positioned in a lower region of the wall or side wall 213 of the humidification chamber 216 above the water level. The inlet 214 may be positioned such that the gases enter the humidification chamber 216 as close as possible to the upper surface of the water 220. The inlet 214 and outlet 218 may be substantially perpendicular or orthogonal to each other. The inlet 214 and outlet 218 may also be positioned at substantially 90 degrees to each other. As warmed and humidified gases swirl i.e. rotate upwards within the humidification chamber 216, injecting the gases as low as possible increases the length of the gases flow path. The gases residence time and/or the flow path length may also be increased for at least some of the gases flow within the chamber.

Alternatively the inlet 214 may be positioned closer to an upper surface of the chamber 216. The inlet 214 is arranged such that gases entering the chamber 216 enter substantially tangential to the side wall. The inlet 214 is positioned such that at least some of the gases attaches to the side wall and travels along the side wall resulting in the spiral i.e. vortex flow being developed in the chamber 216.

Figs. 2A to 2D show alternative humidification chamber configurations that are similar to but modified versions of the chamber shown in Figure 2. In Fig. 2A, a top part 215 of the chamber 216a is frustoconical with the outlet 218a replacing the top of the cone. The remainder of the side wall of the chamber 216a depends down from the frustoconial part 215. While shown as being vertical, other profiles are also possible for the remainder of the side wall. The chamber 216b of Fig. 2B is similar to that of Fig. 2A but the cone forming the upper part of the chamber 216b is truncated to a greater extent. While the top of the chamber 216b extending between the base of the outlet 218b and the top of the frustoconical portion is shown as being flat or parallel to the base of the chamber 216b, other angles of slope are possible including upwards or downwards from the outlet 218b. Further as shown in Fig. 2B, the inlet 214b may have a smaller cross-sectional area than the outlet 218b, the inlet 214b being configured to increase the speed (accelerate) of gases entering the chamber 216b and to promote a jet-like flow. In Fig. 2C, other than the outlet 218c and inlet 214c, the side wall of the chamber 216c is frustoconical and in Fig, 2D, the side wall of the chamber 216d is entirely or substantially entirely dome-shaped apart from at the outlet 218d and inlet 214d.

Similar to Fig. 2, the inlets 214a, 214b, 214c, 214d may be provided above but proximate to the surface of the water inside the humidification chambers 216a, 216b, 216c when filled to their recommended level, although they may be positioned at a higher point in the side wall or in or near the top of the chambers 216, 216a, 216b, 216c, as shown in Fig. 2B. While shown as being generally horizontal, the inlets 214, 214a, 214b, 214c may be inclined upwards or downwards, preferably downwards to direct gases towards the water inside the chambers 216, 216a, 216b, 216c. Further, while the inlets 214, 214a, 214b, 214c may extend perpendicularly to a tangent to the outer surface of the side wall of the chambers 216, 216a, 216b, 216c, respectively, they may also be offset from perpendicular. More preferably, the inlets 214, 214a, 214b, 214c may be configured to promote gases flow that follows the internal contour of the side wall of the chambers 216, 216a, 216b, 216c. For example, the inlets 214, 214a, 214b, 214c may be generally tangentially mounted to the side wall of the chambers 216a, 216b, 216c so as to encourage swirling or vortex generation. The outlets 218, 218a, 218b, 218c while shown as being in the centre top of the chambers 216, 216a, 216b, 216c, they may be otherwise positioned. For example, they may be positioned elsewhere in a top part of the chambers 216, 216a, 216b, 216c or in the side walls thereof, in which case, preferably an upper portion of the side walls.

Further as shown in the top view in Fig. 2E, the humidification chamber 216e may comprise two or more inlets 214e, preferably tangentially disposed in or mounted to a side wall of the chamber 216e when viewed from above, more preferably to portions of the side wall above but proximate to a normal maximum water level inside the chamber 216e. According to this embodiment, the outlet 218e is preferably provided in the centre of the top of the chamber 216e. The plural inlets 214e serve to improve vortex generation and better controls the flow pattern inside the chamber 216e, at least when directing flow in the same rotational direction - anticlockwise in Fig. 2E. The inlets 214e are disposed adjacent the side wall. The inlets 214e are tangentially disposed in or mounted to the side wall, such that the gases from the inlet are introduced substantially tangential to the side wall. Alternatively, one said inlet may be inverted such that at least said two inlets create opposing flows. Such an arrangement may generate greater turbulence and/or mixing, thereby circulating gases within the chamber. At least two of the two or more inlets 214e may be provided at different heights above the water level although all inlets may be at the same height. Such additional inlets may also be provided to other embodiments. The two inlets 214e may improve mixing and can also improve the formation of the vortex.

In another embodiment illustrated in Figs. 3A and 3B, the inlet 314 is positioned in a lower region of the wall or side wall 313 of the humidification chamber 316 above the water level. The inlet 214 and outlet 318 may also be positioned substantially normal to one other, for example at substantially 90 degrees to each other. In addition, the inlet 314 may be angled (e.g. angled down towards the bottom of the chamber 316) such that gases entering the humidification chamber 316 are pushed towards the water 320 therefore applying a force which creates a "bowl effect" in the water 320. Thus the inlet 314 is configured such that the direction of the gases flow as it exits the inlet 314 and enters the chamber 316, is not perpendicular to the side wall 313, and /or is not parallel with the top or base of the chamber 316. This "bowl effect" may increase the length of the gases flow path and/or may also increase the gases residence time.

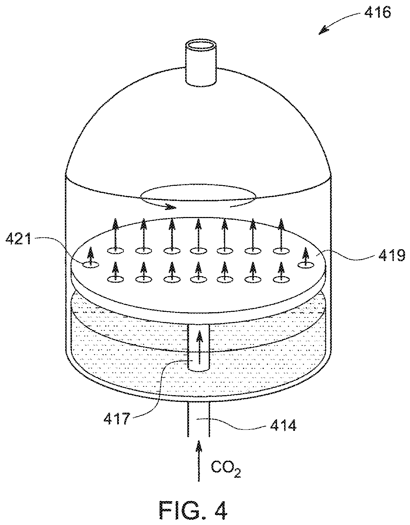

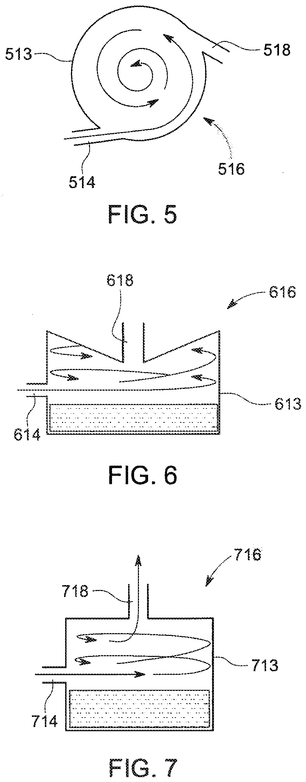

In a further embodiment illustrated in Fig. 4, a rotating diffuser may be provided and disposed within the humidification chamber 416. The rotating diffuser may comprise a shaft 417 coupled or integral with the inlet 414 and a plate 419 connected to the shaft 417. The gases enter the humidification chamber 416 via the inlet 414 and the shaft 417, and are then released via a plurality of openings 421 disposed on the plate 419. The rotating diffuser 415 increases the angular momentum of the gases inside the humidification chamber 416 and thus increases the length of the gases flow path and/or increases the gases residence time. In this example the inlet 414 preferably extends through the base of the chamber 416, coaxially with the central axis of the chamber 416. The plate 419 is a rotating structure that rotates the gases in the chamber 416 to create a vortex. The rotating structure 419 may include cut outs to expose the gases to the water. Fig. 5 illustrates another embodiment where the inlet 514 and the outlet 518 may be positioned in spaced apart positions on the side wall 513. For example, the inlet and outlet are arranged to be co-planar in some embodiments. The inlet and outlet may be substantially parallel. The gases being transported toward the outlet will likely cause turbulence at or adjacent the outlet. This interference and interruption of the gases flow in the chamber at or adjacent the outlet may increase residence time since the gases may "finger" at or adjacent the outlet.

Figs. 6 to 13 illustrate different arrangements for humidification chambers, constructed and operative in accordance with other embodiments. Fig. 6 illustrates an embodiment in which the top of the humidification chamber 616 has a cone shape or an inverted cone shape configured to reflect any upwards moving gases back into the chamber 616. For example, the top may be arranged to form an acute angle relative to the axis of the outlet 618 such that the top of the humidification chamber 616 is angled toward the base to direct the gases towards the center of the chamber. The inlet 614 may be positioned in a lower region of the side wall 613 of the humidification chamber 616 and the outlet 618 on the top (e.g. at the lowest point of the cone). As the warmed and humidified gases swirl upwards in a vortex, the gases may be trapped in an upper section of the humidification chamber 616 corresponding to the highest points of the (inverted) cone-shaped upper wall and then directed towards the center of the chamber 616. This (inverted) cone shape makes it harder for the gases to exit the humidification chamber 616. The inverted cone shape improves humidification performance. The cone shape makes it harder for gases to exit thereby making the gases remain in the chamber to improve the path length and/or improve residence time, thereby resulting in more efficient humidification.

The term 'efficient humidification' should include faster humidification to saturation and/or increasing the amount of humidity into the gases and/or faster saturation at a lower heater plate temperature/power consumption.

In another embodiment illustrated in Fig. 7, the top of the humidification chamber 716 may be flat. The top forms a ceiling that reflects any upwards moving gases back into the humidification chamber 716. Such a configuration increases the length of the gases flow path and/or the gases residence time.

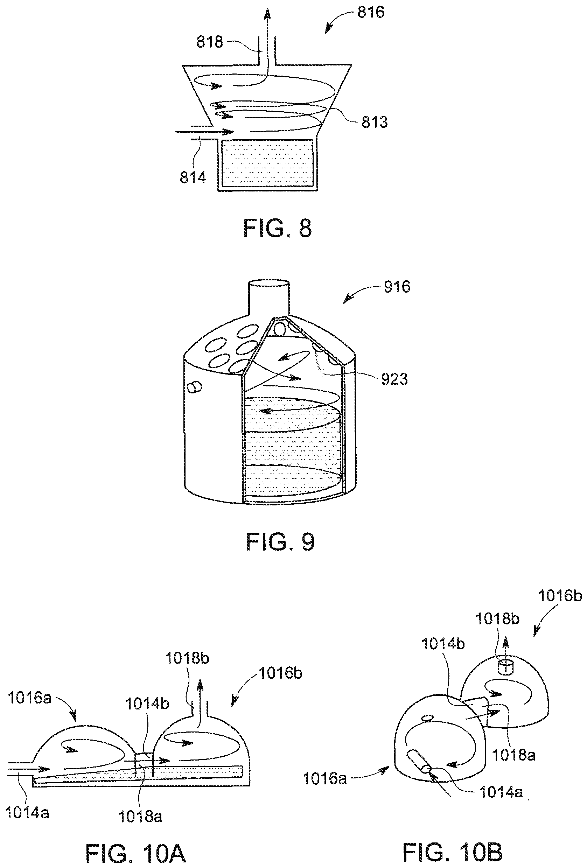

In a further embodiment depicted in Fig. 8, the humidification chamber 816 may be a diffuser shaped chamber having a top shaped as an expanding cone, the radius and/or diameter of the humidification chamber 816 increasing towards the top. In other words, a radius and/or diameter of the humidification chamber 816 adjacent the top is greater than a radius and/or diameter adjacent the inlet 814, the inlet 814 being positioned in a lower region of the side wall 813 of the humidification chamber 816. This configuration of the humidification chamber 816 provides a decrease in the angular velocity of the gases when gases travel closer to the outlet 818. Thus, the gases pressure may change (for example increase) and the travel time of the gases between the inlet 814 and the outlet 818 of the humidification chamber 816, and by extension, the path length and/or residence time may increase.

In one embodiment (see Fig. 9), the humidification chamber 916 has a dome shape i.e. the top of the chamber 916 has a curved profile similar to the one described in relation to Fig. 2. Alternatively, the top of the humidification chamber 916 may be shaped substantially as a nozzle of frustro conical shape when viewed from the side. By nozzle we mean that the effective cross sectional area of the flow path decreases in a direction aligned with the longitudinal axis of the chamber. The dome or nozzle shapes assist in increasing the velocity of the gases as they exit the chamber 916. As a further alternative the inlet may comprise substantially constant internal cross sectional area. As a further alternative the top of the humidification chamber 916 may comprise a cone shape or a trapezoidal prism shape i.e. wherein the top has a smaller width or diameter compared to portions adjacent the upstanding side wall. In addition, protrusions 923 may be provided on an inner surface of the top. The protrusions 923 enable mixing of the gases flow such that the gases flow is more turbulent which, in turn, increases the length of the gases flow path and the residence time. The protrusions cause turbulence of gases by deflecting off the protrusions thereby enabling mixing of the gases flow. The protrusions may be substantially circular or may be dimples. Alternatively the protrusions may be frustroconical or cylindrical or rectangular prism in shape or trapezoid shaped protrusions.

Figs. 10A and 10B illustrate another embodiment in which the humidification chamber comprises two humidification sub-chambers 1016a and 1016b provided in series. A first humidification chamber 1016a may be provided with an inlet 1014a and an outlet 1018a. The first humidification chamber 1016a may be configured such that gases enter the chamber via the inlet 1014a and swirl in a vortex before exiting via the outlet 1018a. A second humidification chamber 1016b may be provided with an inlet 1014b and an outlet 1018b, the inlet 1014b being coupled or integral with the outlet 1018a of the first humidification chamber 1016a. The second humidification chamber 1016a may be configured such that gases enter the chamber via the inlet 1014b and swirl in a vortex before exiting via the outlet 1018b. Providing two humidification chambers and therefore two gases flow paths increases the length of the gases flow path and the residence time.

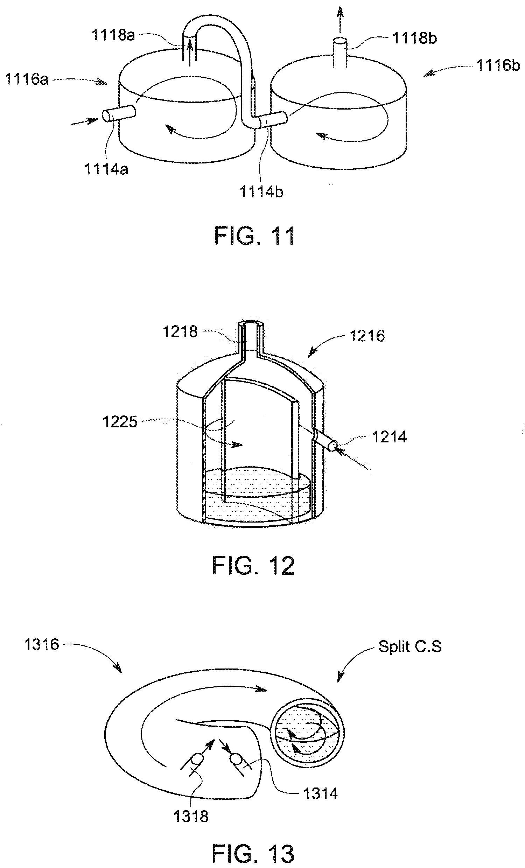

The embodiment of Fig. 11 is similar to the one described in relation to Fig. 10 although the outlet 1118a may be disposed this time on the top of the humidification chamber 1116a and connected to the inlet 1114b of the second humidification chamber 1116b. The first humidification chamber 1116a is configured such that gases enter the chamber via the inlet 1114a and swirl in a vortex before exiting via the outlet 1118a. A second humidification chamber 1116b is provided comprising an inlet 1114b and an outlet 1118b, the inlet 1114b being coupled with the outlet 1118a of the first humidification chamber 1116a. There is a conduit that connects the outlet of the first chamber 1118a to the inlet of the second chamber 1114b. The conduit may be a flexible conduit or may be a rigid conduit. The second humidification chamber 1116a may be configured such that gases enter the chamber via the inlet 1114b and swirl in a vortex before exiting via the outlet 1118b. Providing two humidification chambers and therefore two gases flow paths increases the length of the overall gases flow path and the residence time. It will also be appreciated by those skilled in the art that, although only two humidification chambers are depicted in Figs. 10A-10B and 11, any suitable number of humidification chambers may be provided to increase the length of the gases flow path and therefore the residence time. Connecting multiple chambers to each other may also improve and/or ease the humidifying process as the overall residence time and therefore the duration during which the gases may be in contact with the water surface is increased. The conduit connecting the first and second chambers 1116a, 1116b may be heated to prevent cooling of the humidified gases flowing from the first chamber 1116a to the second chamber 1116b. For example, an electrical heating element, preferably in the form of a wire, may be provided inside, about or within the wall forming the conduit. The conduit may include a thermally insulating sleeve covering all or part of the conduit to thermally insulate the contents of the conduit to prevent or minimise condensation.

In one embodiment illustrated in Fig. 12, one or more internal element(s) 1225 may be provided with the humidification chamber 1216. For example, the one or more internal element(s) may comprise a curved baffle or wall disposed within the gases flow path between the inlet 1214 and the outlet 1218 of the chamber 1216. The baffle or wall may be configured to guide the gases flow within the humidification chamber 1216 before they exit the chamber 1216 at the outlet 1218. In another example, the one or more internal element(s) 1225 may define an internal chamber i.e. the internal element may define an internal volume having an inlet and an outlet. Gases enter the humidification chamber 1216 via the inlet 1214 and are guided along an outer wall of the internal chamber until they enter the internal chamber at an inlet. The gases may be humidified in the humidification chamber 1216 and/or the internal chamber before exiting the internal chamber at an outlet and the chamber 1216 at the outlet 1218. In both configurations, the length of the gases flow path and therefore the gases residence time may be increased as the gases are forced to follow the one or more internal element(s) 1225 before exiting the humidification chamber 1216 at the outlet 1218. Alternatively, gases may via the internal chamber and then pass to the outer chamber 1216.

In a further embodiment depicted on Fig. 13, the humidification chamber 1316 is toroid or donut-shaped and comprises inlet 1314 and outlet 1318 ports positioned on a wall or side wall. The inlet 1314 may be disposed on the humidification chamber 1316 such that the gases swirl in a vortex in a vertical plane along the length of the chamber. The outlet 1318 may be disposed on the humidification chamber 1316 adjacent to the inlet 1314 such that the gases swirl around the entire length of the donut before exiting the chamber.

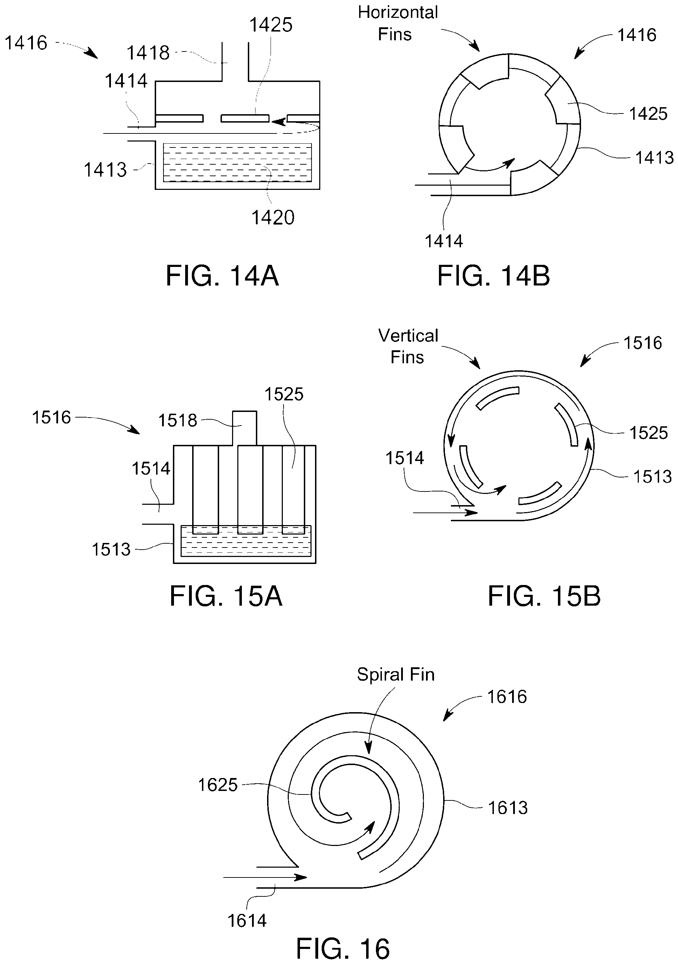

Figs. 14A to 16 illustrate different embodiments in which the humidification chamber includes internal elements configured to guide the gases. In these embodiments, the humidification chambers 1416, 1516, 1616 are provided with an inlet 1414, 1514, 1614 disposed in a lower region of a side wall 1413, 1513, 1613 and an outlet 1418, 1518 disposed at the top (the outlet not being shown in Fig. 16 but preferably similarly disposed at a centre, top of the chamber 1616). The humidification chambers 1416, 1516, 1616 further comprise one or more internal element(s) 1425, 1525, 1625 (e.g. fins or baffles or protuberances) disposed on the inner surface of the side wall 1413, 1513, 1613.

In the embodiment of Figs. 14A and 14B, a plurality of internal elements 1425 may be provided and disposed on the inner surface of the side wall 1413. The internal elements 1425 extend radially from the (inner surface of the) side wall 1413 towards the center of the humidification chamber 1416 and are substantially parallel to the water surface. As previously described in relation to the above embodiments, the humidification chamber 1416 and the inlet 1414 are arranged such that gases entering the humidification chamber 1416 at the inlet 1414 swirl in a vortex within the chamber, passing through gaps provided between the plurality of elements 1425 before exiting via the outlet 1418. In addition, the plurality of elements 1415 may be disposed adjacent to the water 1420 and above the inlet 1414. Such a configuration further increases the gases residence time as the gases are maintained in the lower section of the humidification chamber 1416 by the plurality of elements 1415 for a longer period. As will be appreciated, the internal elements 1425 may be spaced away from the side wall 1413, for example, by mounting the internal elements 1425 in a web or lattice, or other support structure.

The embodiment illustrated in Fig. 15 is similar to that of Fig. 14 but the internal elements 1525 are disposed on or mounted to the inner surface of the top of the chamber 1516. Again, a support structure may be used to mount the internal elements 1525 such that they are spaced apart from the supporting wall of the chamber. The internal elements 1525 extend downwards from or near the top of the chamber 1516 towards the center or bottom of the humidification chamber 1516, preferably substantially perpendicular to the water surface. Alternatively, the internal elements 1525 may be disposed on or mounted to the inner surface of the base. The internal elements 1525 may extend from the (inner surface) of the base towards the center or top of the humidification chamber 1516 and may be substantially perpendicular to the water surface. Both configurations further increase the gases residence time as the gases are maintained in the peripheral section of the humidification chamber 1516 (i.e. between the wall or side wall 1513 and the plurality of elements 1525 as depicted by the arrows on Fig. 15) by the plurality of elements 1525, retaining the gases in the chamber 1516 for a longer period.

Fig. 16 shows another embodiment in which the internal elements 1625 comprises a single spiral fin provided to guide the gases flow within the humidification chamber 1616. In such configuration, the length of the gases flow path is determined by the spiral and can be elongated. The gases residence time is increased as the gases are forced to follow the spiral fin before exiting the humidification chamber 1616 at the outlet. Similar to the embodiment of Figs 15A and 15B, the spiral fin 1625 may be disposed on or mounted to the top and/or bottom of the chamber 1616, or otherwise held in position in the chamber 1616 using a support structure.

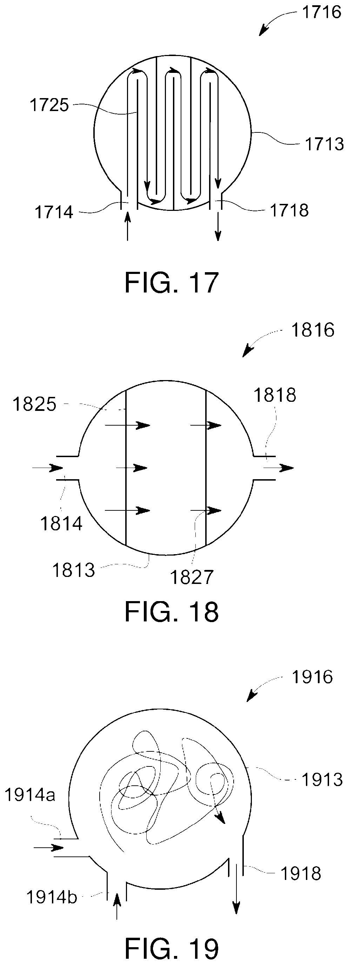

Figs. 17 to 19 illustrate different arrangements for a humidification chamber, constructed and operative in accordance with further embodiments. In one embodiment as shown in Fig. 17, a plurality of internal elements 1725 are provided and disposed on the inner surface of the wall or side wall 1713. The internal elements 1725 preferably extend from the inner surface of the side wall 1713 towards the opposing wall of the chamber 1716, substantially perpendicular to the water surface. As shown, the internal elements 1725 are preferably substantially parallel. The internal elements 1725 are advantageously configured and/or disposed so as to form a winding or serpentine path for the gases flow. The gases entering the humidification chamber 1716 via the inlet 1714 are forced to pass through the winding path before exiting the chamber at the outlet 1718 positioned on the wall or side wall 1713. This configuration increases the length of the gases flow path and therefore the gases residence time. As will be appreciated, the internal elements 1725 may additionally or alternatively be disposed on or mounted to the top and/or bottom wall of the chamber 1716, or otherwise held in the chamber 1716 via a support structure.

Fig. 18 shows a further embodiment in which two or more internal elements 1825 may be disposed within the gases flow path between the inlet 1814 and the outlet 1818 of the humidification chamber 1816. Preferably, as shown, both the inlet 1814 and outlet 1818 are positioned on the wall or side wall 1813. However, as with other embodiments, at least the outlet 1818 may be positioned at or near a top of the chamber 1816, including offset from the centre thereof. For example, the inlet 1814 could be positioned at a side wall of the chamber 1816 near but above the water level, with the outlet 1818 provided in the top of the chamber 1816 at point laterally spaced apart from the inlet 1814 such that the outlet 1818 is not positioned directly above the inlet 1814. The inlet 1814 may also be positioned in the top wall of the chamber 1816, preferably adjacent the side wall. Preferably, the inlet 1814 and outlet 1818 are provided on or adjacent substantially opposing parts of the side wall. The two or more internal elements 1825 may be walls attached to opposite sides of the inner surface of the wall or side wall 1813 and spanning the entire humidification chamber widths at the points they are provided. A plurality of openings 1827 of different sizes and/or shapes may be provided on the internal elements 1825. The dimensions and locations of the openings 1827 may be selected so as to control the gases flow and increase the gases residence time within the humidification chamber 1816.

In a further embodiment, the humidification chamber 1916 may comprise a plurality of inlets 1914a and 1914b as illustrated in Fig. 19. The inlets 1914a and 1914b may be positioned on the wall or side wall 1913 such that the gases flow entering the chamber via the first inlet (e.g. 1914a) interact with the gases flow entering the chamber 1916 via the second inlet (e.g. 1914b) thereby creating a turbulent gases flow. The mixing of the gases through the two inlets 1914a, 1914b causes turbulent flow within the chamber 1916. The gases velocity into the inlets 1914a, 1914b may be the same or one inlet may include flow restricting elements to change the velocity of the gases being introduced into the chamber 1916. The inlets 1914a, 1914b may be angled relative to each other. In one form the inlets 1914a, 1914b may be arranged such that one inlet is normal to the other inlet. Alternatively, the inlets 1914a, 1914b may be arranged around the periphery of the chamber 1916 at any suitable angle or at any suitable peripheral position relative to each other . This configuration enables the gases flows to remain longer within the humidification chamber 1916 and therefore increases the gases residence time. Two or more of the inlets 1914a, 1914b may receive gases from the same gases source. At least one of the inlets 1914a, 1914b may receive gases from a gases source different to the gases source of at least one other of the inlets 1914a, 1914b. Different or the same gases may be provided by different gases sources. For example, for applications to respiratory therapy, ambient air may act as a first gases source with an oxygen cylinder being used as a second source to provide oxygen enriched gases to a patient.

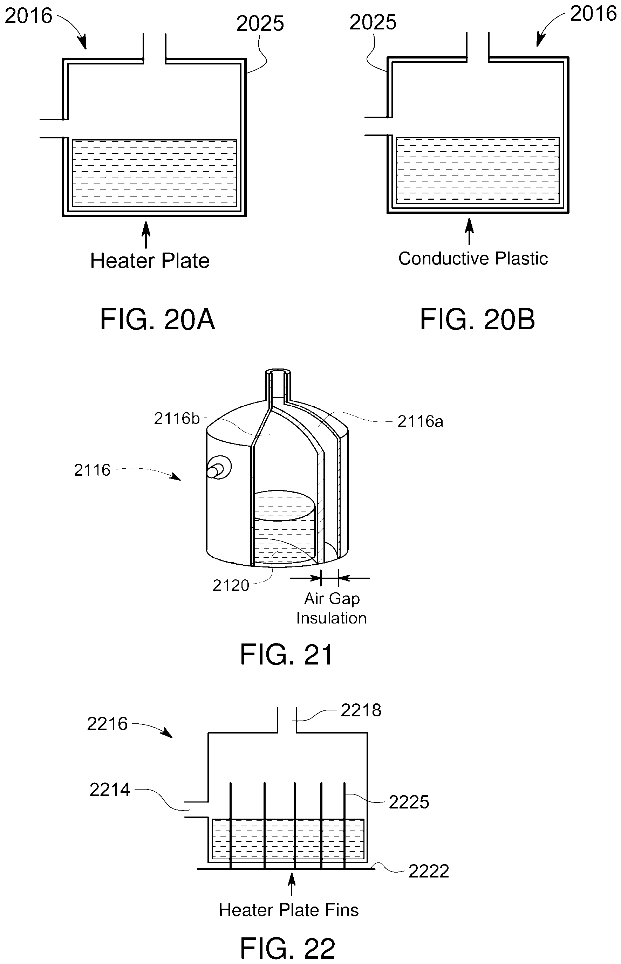

Reference is now made to Figs. 20 to 31, which are examples of humidification chambers, constructed and operative in accordance with other embodiments. The humidification chambers depicted in Figs. 20 to 31 increase the efficiency of a humidification chamber by increasing the surface area available for heat transfer and/or mass transfer, and/or improving the heat transfer and/or mass transfer.

Figs. 20 and 21 illustrate different arrangements for a humidification chamber, in which the, or an additional heating element 2025 for heating the water in the chamber, or a heat conductive medium is provided to more than just the base of the chamber 2016. For example, as shown in Fig. 20A, the chamber heater plate may extend up at least a portion of the side walls of the chamber 2016 and/or be provided at the top of the chamber 2016. Alternatively, as shown in Fig. 20B, a heat conductive medium may be provided to at least a portion of the chamber wall in additional to the part of the chamber in direct contact with the chamber heater (usually the base of the chamber 2016). Thus an additional heating element and/or heat conductive plastic casing may be provided and disposed around the humidification chamber 2016. The entire chamber 2016 may be heated using the heater element 2025 enclosing the chamber 2016. These configurations increase the heat transfer and/or mass transfer to the gases by providing a further heating element 2025 (in addition to the heater plate 122 on Fig. 1) and preventing any heat to escape the humidification chamber 2016. Additionally or alternatively, part or all of the chamber walls may be formed by the heater and/or the heat conductive medium. The use of a heat conductive medium beyond the boundaries of the chamber in contact with the heater plate serves to promote heat transfer and/or mass transfer to the chamber contents due to the conductive heat transfer and/or mass transfer enlarging the part of the chamber heated by conduction. The heater element may be a heater plate or a heater wire wrapped around part of, or a portion of, the humidification chamber or PTC sheet or PTC material wrapped/disposed about the humidification chamber.

Fig. 21 illustrates another embodiment where the humidification chamber 2116 comprises two internal chambers 2116a and 2116b. A first chamber (e.g. 2116a) may be adapted to surround a second chamber (e.g. 2116b). The second chamber may be adapted to receive and hold a volume of water 2120. Thus, the first chamber 2116a creates an air gap about the second chamber 2116b, preventing heat loss in the second chamber 2116b. The air gap between the first and second chambers 2116a, 2116b acts as an insulation layer to reduce heat loss. In some optional configurations a thermal insulating material e.g. a foam or polystyrene or any other suitable thermal insulator may be located within the air gap.

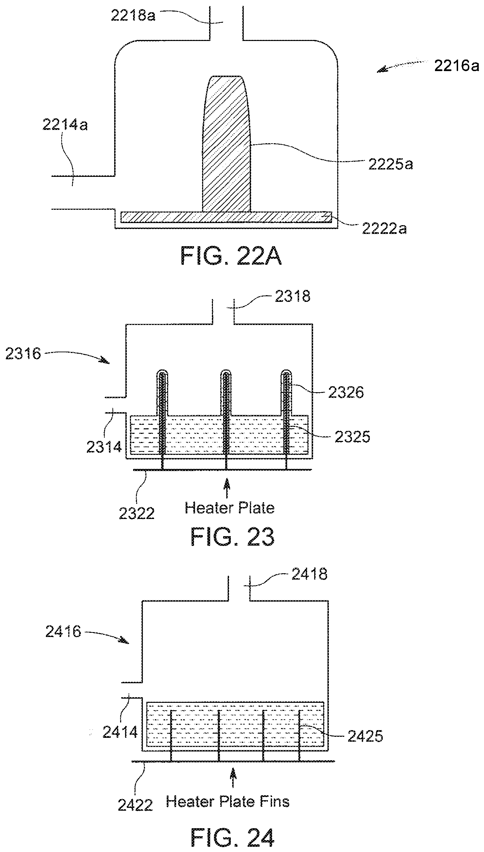

Figs. 22 to 28 illustrate additional embodiments in which the efficiency of a humidification chamber is increased by increasing the surface area available for heat transfer and/or mass transfer.

Fig. 22 shows a humidification chamber 2216 comprising a heater plate 2222 which is provided with a plurality of heating elements 2225. The heating elements 2225 (e.g. fins) may extend upwards from the surface of the heater plate 2222 towards but preferably spaced apart from the top of the humidification chamber 2216. The heating elements 2225 may be arranged such that at least a portion is disposed above the water level thereby providing a further heat source to the gases present in the humidification chamber 2216. In addition, the heating elements 2225 are provided in part in the water, thereby providing an increased surface area of the heater available for heat transfer and/or mass transfer to the water. The upstanding heater plate fins 2225 may comprise a substantially rectangular profile or shape to increase the surface area of the heater. Additionally or alternatively, they may have an arcuate cross-section i.e. curved when viewed from above. Additionally or alternatively, the fins 2225 may be configured and/or positioned so as to promote a desired gases flow inside the chamber 2216. For example, the fins 2225 may create a tortuous path for the gases to as to increase residence time in the chamber 2216.

Fig. 22A illustrates an embodiment of a chamber similar to that depicted in Fig. 22 but rather than having a plurality of heater plate fins 2225, a heated column 2225a extends up from the heater plate 2222a. The heated column 2225a may be in the form of a substantially rigid heater element or a flexible element or conductive track provided to a support member. The heater element or support member may be structurally connected to the heater plate 2222a or pass through an opening therein. The heater element of the column 2225a may be electrically connected to the heater plate 2222a such that a single power source supplies power to both heaters or a separate connection may be provided therefor. Similar to the heated fins 2225, the conductive column 2225a provides additional heating to the water and also directly to the gases inside the chamber 2216a. Further, the column 2225a aids in controlling the swirling flow of gases about the chamber 2216a by removing or reducing turbulent flow at the centre of the vortex, reducing contact between gases flows that are opposing to one another.

Fig. 23 illustrates an embodiment similar to the one depicted in Fig. 22. However, in this embodiment, the heating elements 2325 may be provided with a layer 2326 having hydrophilic properties such that a thin layer of water is encouraged to cover the portion of the heating elements 2325 disposed above the water level. Thus, the surface area for heat transfer and/or mass transfer to the water is further increased. The hydrophilic layer 2326 attracts water across its surface, forming a thin layer of water which is more quickly and easily vaporized than water in the form of a reservoir heated by a heater plate 2322 positioned under the reservoir, at least during initial use. The hydrophilic layer 2326 can also help to increase the surface area of the water that is heated. The hydrophilic layer 2326 may be positioned across a portion of the chamber 2316 and may extend across the chamber 2316, as shown in the illustrated configuration of Fig. 23. In an alternative configuration each heater plate element e.g. heater plate fin 2325 may include a coating of hydrophilic material disposed thereon. The hydrophilic material can draw water towards and about the heater plate elements 2325, thereby improving contact between the heater plate elements 2325 and water to improve heating/vaporization of the water.

In a further embodiment the heater plate may comprise wicking structures or a wicking coating on the fins of the heater to draw water from the reservoir such that water extends and covers the entire fin. The structures may be microstructures or capillary tubes or other wicking structures that draw water along the heater fins.

Fig. 24 illustrates another embodiment similar to those shown in Figs. 22 and 23. In the embodiment of Fig. 24, the heating elements 2425 do not comprise a portion disposed above the water level. However, the heating elements 2425 still provide an increased surface available for heat transfer and/or mass transfer to the water.

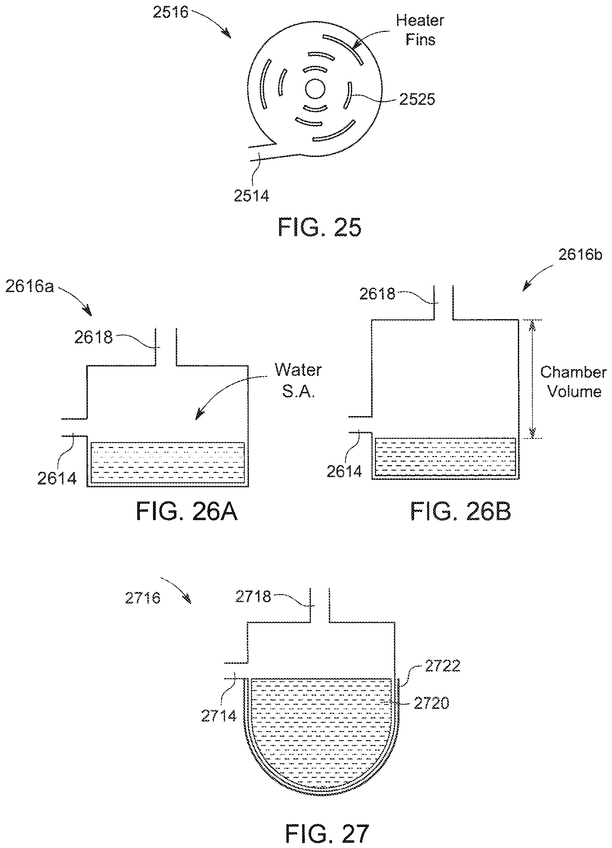

Fig. 25 illustrates a further embodiment similar to those shown in Figs. 22-24. Instead of being arranged in a linear arrangement, the heating elements of Fig. 25 may be curved heater fins disposed within the gases flow path between the inlet 2514 and the outlet (not shown) of the humidification chamber 2516. This alternative configuration promotes circular flow within the chamber 2516 and further increases the gases residence time. The fins 2525 may extend below the normal water level in use (similar to Fig 24) or above the normal water level in use (similar to Figs. 22 and 23).

Figs. 26A and 26B illustrate two humidification chambers 2616a, 2616b having different sizes wherein the chamber volume available for gases is greater in the humidification chamber 2616b of Fig. 26B than in the humidification chamber 2616a of Fig. 26A. In the embodiment depicted in Fig. 26A, the chamber volume available for gases is reduced therefore increasing a ratio of water surface available for gases heating compared to the chamber volume, or at least the headspace above the water inside the chamber. This has a shorter residence time, but a larger surface to volume ratio for the water. This reduces heat loss through the chamber walls, thereby reducing chances of condensation. Thus, the efficiency of the humidification chamber 2616a is increased by providing a more efficient heat transfer and/or mass transfer. Increasing the surface area of the water relative to the gas volume increases the amount of gas that contacts the water. This coupled with the vortex flow of gases due to the inlet being tangential to the side wall can improve the efficiency of humidification. For example gases can be humidified faster or a lower power heater may be used. As an alternative, the width/diameter of the chamber can be increased in order to increase the surface area of water available for humidification.

Fig. 27 shows a humidification chamber 2716 comprising a bottom wall having a bowl shape. In addition, a heater plate 2722 having a corresponding bowl shape is provided to heat the humidification chamber 2716. This configuration provides an increased surface area for heat transfer and/or mass transfer from the heater plate 2722 to the water 2720.

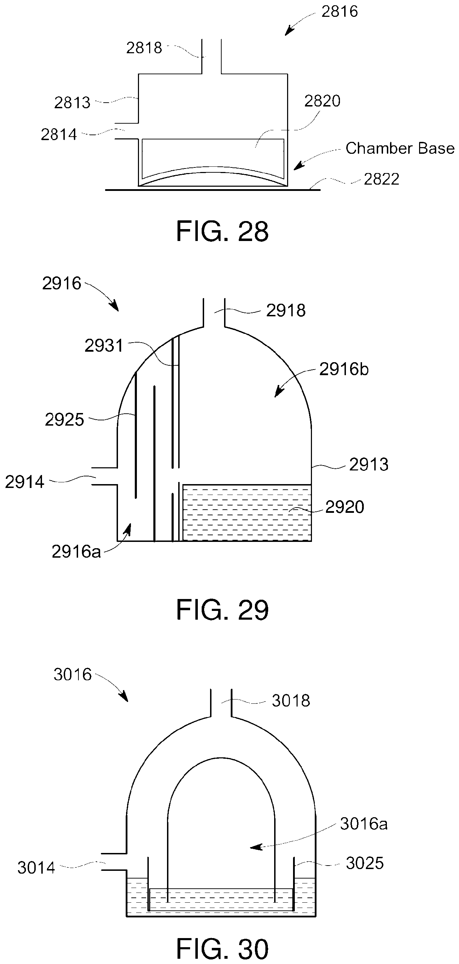

Fig. 28 illustrates another embodiment in which the bottom wall or base of the humidification chamber 2816 comprises a varying thickness. For example, the central portion of the bottom wall or base may be configured to have an increased thickness compared to the portions adjacent the side wall or wall 2813. The increased thickness section may comprise a conductive material to improve heat transfer and/or mass transfer to the water. Fig. 28 shows this particular arrangement where the outer surface of the bottom wall or base is flat and the inner surface is curved such that the base has a varying thickness. This configuration provides an increased surface area for heat transfer and/or mass transfer from the heater 2822 to the water 2820 and also a better heating of the central mass of the water such that heat may be transferred faster to the entire volume of water 2820.

Fig. 29 shows a humidification chamber 2916 comprising a first chamber 2916a and a second chamber 2916b separated by an internal wall 2931. Gases enter the humidification chamber 2916 via the inlet 2914 of the first chamber 2916a and are guided to the second chamber 2916b by a plurality of heating elements 2925 positioned within the first chamber 2916a to form a controlled gases flow path. The plurality of heating elements 2925 may be, for example, fins configured to heat the gases prior to humidification. Further, the plurality of internal elements 2925 may be disposed so as to form a winding or serpentine path for the gases flow. The heating elements 2925 may extend radially from the inner surface of the top and/or base and/or side wall 2913 of the chamber 2916 and may have any suitable shape and/or configuration e.g. rectangular, curved, openings of different dimensions and/or shapes, etc. The second chamber 2916b may be adapted to hold a volume of water 2920, which can be heated by a heater plate (not shown). Heated gases enter the second chamber 2916b via an outlet of the first chamber 2916a and are humidified before exiting via the outlet 2918. In addition, the outlet of the first chamber 2916a may be configured to introduce the heated gases to the second chamber 2916b at a direction substantially tangential to the internal wall 2931 such that gases swirl in a vortex as they exit the second chamber 2916b via the outlet 2918. Pre-heating the gases can increase the "capacity" of the gases to collect an increased amount of humidity. The heating elements may be plate heaters that are disposed in the chamber. The heaters may be supplied with power by a wired connection that is coupled to a heater connector (not shown).

Fig. 30 shows a humidification chamber 3016 comprising one or more internal element(s) 3025 disposed so as to allow an internal chamber 3016a to be disposed therein. Gases enter the chamber 3016 via the inlet 3014 and at least a portion of the gases flow may flow around the outer surface of the internal chamber 3016a before exiting the chamber 3016 via the outlet 3018. Another portion of the gases flow may enter the internal chamber 3016a via an inlet (not shown) and exit the internal chamber 3016a via an outlet (not shown) prior to exiting the chamber 3016 via the outlet 3018. Providing an internal chamber within a humidification chamber increases the gases flow path and therefore the gases residence time for at least a portion of the gases flow. The increased velocity and/or the vortex flow in the inner chamber increases the path length of the gases in the chamber.

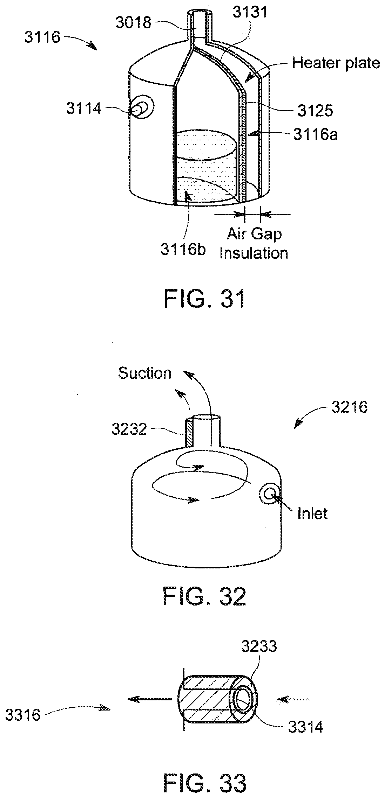

Fig. 31 illustrates another embodiment where the humidification chamber 3116 comprises two internal chambers 3116a and 3116b. A first chamber (e.g. 3116a) may be adapted to surround a second chamber (e.g. 3116b) adapted to receive and hold a volume of water 3120. Thus, the first chamber 3116a creates an air gap preventing heat loss in the second chamber 3116b. The air gap is between the inner chamber and outer chamber. The air gap is between the second heater plate and the outer chamber.

In addition, an additional heating element 3125 may be provided to heat an internal wall 3131 separating the two chambers thereby further preventing heat loss in the humidification chamber 3116. In use, gases entering the first chamber 3116a via the inlet 3114 will be heated by the heating element 3125 before entering the second chamber 3116b. Heated gases enter the second chamber 3116b via an outlet of the first chamber 3116a and are humidified before exiting via the outlet 3118. In addition, the outlet of the first chamber 3116a may be configured to introduce the heated gases to the second chamber 3116b at a direction substantially tangential to the internal wall 3131 such that gases swirl in a vortex as they exit the second chamber 3116b via the outlet 3118.

Reference is now made to Figs. 32 to 35, which are examples of humidification chambers, constructed and operative in accordance with other embodiments. The humidification chambers depicted in Figs. 32 to 35 increase the efficiency of a humidification chamber by increasing the velocity of the gases.

Fig. 32 illustrates a humidification chamber 3216 comprising at least one suction outlet 3232. In use, the suction outlet 3232 may be configured to suck the gases out of the humidification chamber 3216 such that the velocity of the gases is increased. This arrangement increases the velocity of the gases present in and exiting the humidification chamber 3216. The suction outlet 3232 may be coupled to a vacuum or pump that creates a negative pressure to cause suction.

Fig. 33 depicts an inlet 3314 of a humidification chamber 3316 comprising a heated nozzle 3333. The heated nozzle 3333 may be configured to cause the gases to expand as they pass through into the humidification chamber 3316. This arrangement alters and/or changes (e.g. increases or decreases) the velocity of the gases entering the humidification chamber 3316.

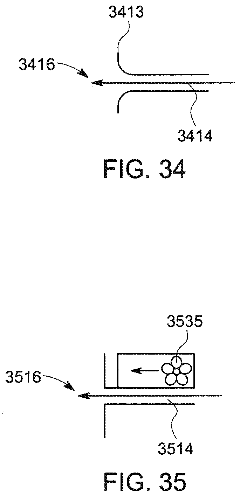

Fig. 34 illustrates another embodiment in which the transition between an inlet 3414 and the interior of the humidification chamber 3416 may be configured to reduce the pressure drop. For example, but not limited to, the portion of the nozzle inlet 3414 adjacent to the side wall or wall 3413 of the humidification chamber 3416 may be curved so as to ensure a smooth transition for the gases entering the chamber 3416.

Fig. 35 shows another embodiment of a humidification chamber 3516 with an inlet 3514 comprising a nozzle blower 3535. The blower 3535 may be dimensioned so as to be positioned within, around or adjacent the inlet 3514. In use, the blower may be configured to accelerate the gases passing through the inlet 3514 and entering the chamber 3516. The nozzle blower 3535 may be a small turbine or a fan. The nozzle blower 3535 can be powered by power transmission lines that may be routed through a supply tube and a connector of the supply tube.

In other embodiments (not shown), the velocity of the gases may be increased by providing a small inlet area, and/or reducing the friction forces inside the chamber. The inlet may be, for example, a nozzle having a taper configured to decrease in diameter so as to speed up and focus the gases flow entering the humidification chamber, that is, to create a jet flow of gases into the chamber. The jet flow of gases causes the gases to attach to the chamber. In other words, the inlet may be provided as a substantially tubular body having a first diameter distal from the humidification chamber or the side wall of the humidification chamber greater than a second diameter proximal to the humidification chamber or side wall of the humidification chamber. In addition, and/or alternatively, an inner diameter of the inlet may be less than an inner diameter of the outlet. Additionally, and/or alternatively, a particular value for a ratio between the inner diameter of the inlet and the diameter of the bottom wall of the humidification chamber may be defined. In one embodiment, the length of the inlet may be of approximately 32 mm and the inner diameter of the inlet may be of 5 mm with a 1.5 degree taper opening up into the humidification chamber. The length of the outlet may be of approximately 20.5 mm and the inner diameter of the outlet may be of 19 mm with a 1.5 degree taper opening up into the humidification chamber. Reducing the friction forces inside the chamber may be achieved by processing, for example, the inner surface of the humidification chamber so as to remove any asperities. In another example, the inner surface of the humidification chamber may be processed so as to add recesses and/or dimples to reduce the resistance to flow.

It will be apparent to those skilled in the art that the embodiments described in the previous paragraph and in relation with Figs. 32 to 35 may be combined with any suitable embodiment described in relation with Figs. 1 to 31. Further, while the chamber inlets (and/or outlets) are generally shown extending from a position outside of the chambers to a wall forming the chamber, it will be appreciated that the inlets (and correspondingly the outlets) may extend from a point outside of the chamber or the chamber wall to a position inside the chamber. Further, in many embodiments, the inlets and outlets may simply be formed by an aperture in a wall of the chamber.

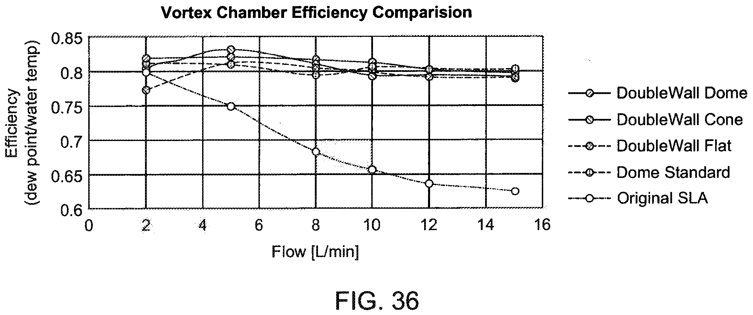

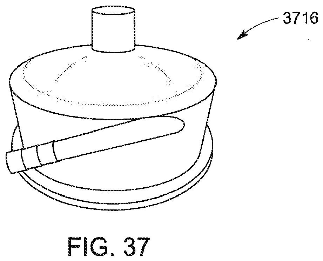

Reference is now made to Fig. 36, which is a graph illustrating the efficiency at different flow rates of humidification chambers, constructed and operative in accordance with embodiments disclosed herein. The different humidification chambers are illustrated in Figs. 37, 38, 39, and 40. For comparison, efficiency at different flow rates of Fisher & Paykel SH870 surgical humidification system humidification chamber (Fig. 41) is also presented.

Fig. 37 illustrates a humidification chamber 3716, constructed and operative in accordance with an embodiment. The top of the humidification chamber 3716 has a dome shape similar to the ones described in relation to Figs. 1 and 9 i.e. it is generally convex when viewed from outside of the chamber. In addition, the chamber 3716 is provided with an outer layer to provide insulation.

Fig. 38 illustrates a humidification chamber 3816, constructed and operative in accordance with another embodiment. The top of the humidification chamber 3816 has an inverted cone shape similar to the one shown in Fig. 6. In addition, the chamber 3816 is provided with an outer layer to provide insulation.

Fig. 39 illustrates a humidification chamber 3916, constructed and operative in accordance with a further embodiment. The top of the humidification chamber 3916 is flat (similar to the chamber shown in Fig. 7) and an outer layer is provided to provide insulation.

Fig. 40 illustrates a humidification chamber 4016, constructed and operative in accordance with another embodiment. The top of the humidification chamber 4016 has a dome shape similar to those shown in, for example, Figs. 2 and 37. However, by contrast to Fig. 37, no outer layer for insulation is provided.

Fig. 41 illustrates a humidification chamber 4116 currently being sold and used as part of the SH870 surgical humidification system from Fisher & Paykel Healthcare Limited.

Measurement of dew point was carried out for the different chambers at different flow rates. Gases were supplied to the inlet of the different chambers at a constant rate. The constant rate was a predefined flow rate and all the chambers were supplied with the same flow rate of gases. The chambers were heated in a customized Fisher & Paykel Humigard® system which allowed the heater plate temperature to be controlled by the user. The Humigard heater base essentially consists of a sprung heater plate that is provide with a rail on each side thereof. The chamber has a rim near its base which is received underneath the rails. The sprung heater plate pushes upwards into the base of the chamber to ensure good thermal contact. A sprung bar is provided at the entrance for the chamber to the rails so as to lock the chamber in the operative position. Pushing down on the bar enables insertion / removal of the chamber.

The outlet of the chamber was connected to a hygrometer (used for testing purposes in a test rig) which measured the dew point of the gases at an end of a heated insufflation tube. In addition, thermostats were used to measure the temperature of the water, the gases leaving the chamber, and the external environment or ambient conditions. The efficiency was then calculated by dividing the dew point temperature by the water temperature as illustrated in the following equation:

$$Efficiency \ = \ \frac{Dew \ Point \ (°C)}{Water \ Temperature \ (°C)}$$

The results are summarized below in Table 1.

Table 1 - Efficiency for the different humidification chambers at different flow rates

| Flow (L/min) | Fig. 41 | | Fig. 37 | | Fig. 38 | | Fig. 39 | | Fig. 40 | |
|---|---|---|---|---|---|---|---|---|---|---|
| | Dew point | Water temp. | Dew point | Water temp. | Dew point | Water temp. | Dew point | Water temp. | Dew point | Water temp. |
| 2 | 33.4 | 41.8 | 34.2 | 41.8 | 34 | 42.3 | 32.7 | 42.4 | 33.5 | 41.3 |
| 5 | 31.1 | 41.6 | 33.8 | 41.2 | 34.6 | 41.6 | 33.8 | 41.6 | 33.4 | 41.2 |
| 8 | 33.8 | 40.8 | 33.9 | 41.7 | 33.7 | 41.5 | 33.4 | 41.5 | 33 | 41.6 |
| 10 | 27.2 | 41.4 | 33.7 | 41.4 | 33 | 41.6 | 33.2 | 41.6 | 33.4 | 41.6 |
| 12 | 26.5 | 41.5 | 33.4 | 41.5 | 33.2 | 41.8 | 33 | 41.8 | 33.3 | 41.4 |
| 15 | 25.6 | 41 | 33.3 | 41.7 | 33.2 | 42 | 32.8 | 41.4 | 33.4 | 41.5 |

As seen in the table above and in Fig. 36, the efficiencies of the humidification chambers 3716, 3816, 3916, and 4016 according to embodiments of the disclosure are substantially similar while the efficiency of the humidification chamber 4116 decreases markedly in comparison as flow rate increases. Having a substantially flat efficiency response is desirable and achieved in all the vortex humidification chambers of Figs. 37-40. All the chambers 3716, 3816, 3916, and 4016 perform slightly differently although some chambers (e.g. humidification chamber 3716) have a slightly more stable efficiency response. A vortex humidification chamber as used herein is a chamber in which spiral flow i.e. vortex flow is established. The gases within the chamber flow in a vortex due to the orientation of the inlet relative to the side wall. The increased efficiency is due to the vortex flow achieved in the chamber.

The embodiments of the humidification chamber are described with reference to use in surgical humidification applications such as, for example, but not limited to, humidification of insufflation gases. For surgical humidification, a flow rate of between 2 L/min to 20 L/min, preferably between 8 L/min to 15 L/min has been found to provide an improved humidification efficiency of the gases, for the chamber of Figure 38. In other examples a flow rate of less than 2L/min is envisaged. The inlet may be shaped and structured such that the minimum flow rate can be lowered or increased. The improved humidification efficiency is advantageous because when humidifying to the same humidity set point, it requires less power to heat the water in the chamber (compared to conventional chambers, e.g. the SH870 chamber of Fig. 41) due to the movement of the gases within the chamber. The movement of the gases may also cause the water in the chamber to move, thus further reducing the power required to heat the water due to the movement of the water. The improved efficiency may also result in a substantially flat humidification response for the operational flow range of the humidification chamber (and humidification system), wherein one example operational flow range is between 2 L/min and 20 L/min. The improved humidification efficiency results in reduced power requirements and an improved humidity delivery to patients over a wider flow range. The improved efficiency may also mean faster humidification of a volume of gases. Different treatments may require different flow rate ranges. For example, high flow treatment via a nasal cannula may typically involves flow rates of between about 20 L/min and about 120 L/min and between about 20 L/min and about 90 L/min, preferably about 40 L/min and about 70 L/min in anesthesia (includes sedation) applications. Thus the minimum useful flow rate is about 2 L/min for the chamber of Fig. 37. The chamber and/or its components (e.g. inlet) can be shaped, structured and/or configured depending on its application and the applicable flow ranges.

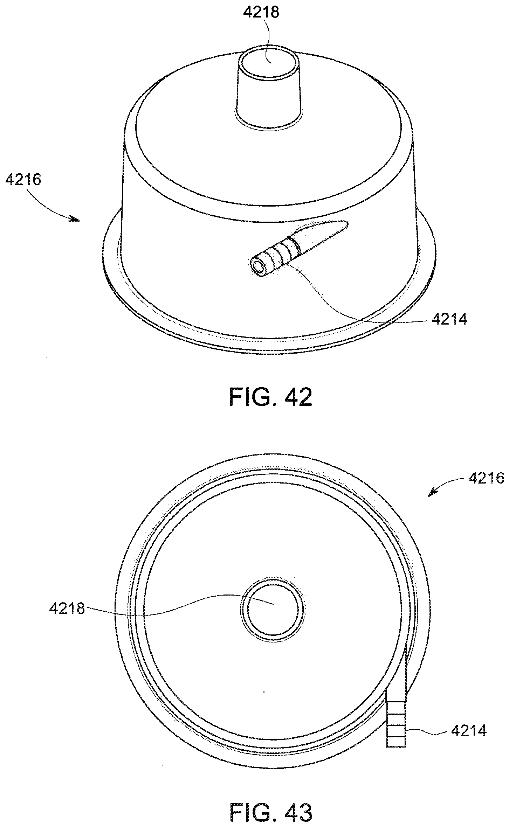

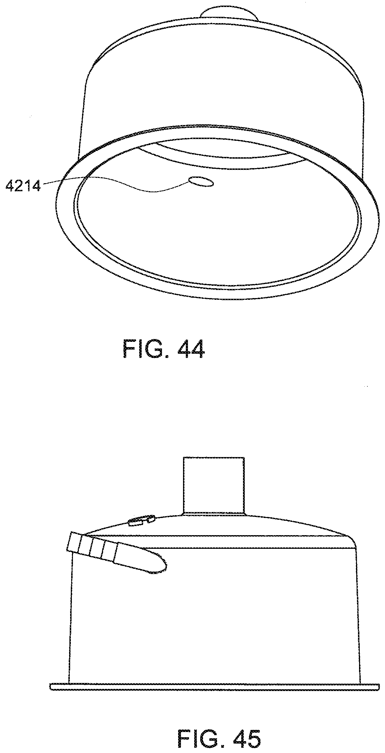

Figs. 42 to 44 show a humidification chamber 4216 constructed according to an embodiment. This embodiment is the same or similar to the chamber 4016 depicted in Fig. 40. As shown in Figs. 42 and 43, the inlet 4214 is provided to the side wall of the chamber 4216 such that it extends tangentially therefrom. Further, the inlet 4214 preferably has a much smaller cross-sectional area than the outlet 4218, as shown. The tangential mounting promotes spiral flow which is aided by the narrow conduit forming the inlet 4214, this causing the gases flow to form a jet or jet like flow and increase velocity of the gases entering the humidification chamber 4216. The gases exiting the inlet 4214 are in the form of a jet, that is, a jet of gases enters the humidity chamber through the inlet. The inlet 4214 is shaped and configured to cause a rapid stream of fluid to be forced out of a small opening, that is, to accelerate the flow of gases through the inlet 4214. According to preferred embodiments, the outlet 4218 has a diameter in the range of 15 mm to 30 mm, more preferably 20 mm to 25 mm and most preferably about 22 mm. The inlet 4214 preferably has a diameter in the range of 3 mm to 10 mm, more preferably 3 mm to 6 mm and is most preferably about 4 mm. While the outlet of the inlet 4214 may be circular, it may be other shapes. For example, it may be elliptical as shown in Fig. 44. The inlet diameters are example diameters. The inlet diameter is related to the inlet flow rate, as diameter increases flow rate into the chamber. The inlet diameter provided for Fig 44, are for a flow rate of up to 70L/min. The higher velocity gases flow causes the gases to attach to the side wall and follow the contour of the side wall. A bulk or average gases flow introduced into the chamber attaches to the side wall and is imparted a rotational component e.g. a rotational inertia that causes the gases to spiral within the chamber. The rotation of the gases flow in the chamber increases the gas flow path length of the gas flow i.e. the gas flow path length is increased for the bulk gases flow. This is because the bulk gases flow rotates several times within the chamber thereby crossing a greater distance over the water i.e. the increased path length causes the gases to exposed to a greater length of the water by circling the water several times. This can provide improved humidity. To improve ease of manufacture, the inlet 4214 may be formed separate from the chamber 4216 and permanently or releasably joined thereto. Any standard means of sealably joining the inlet 4214 to the chamber 4216 may be used, such as an interference fit, threaded engagement, adhesives, vibration and other forms of welding etc).

The conduit forming the inlet 4214 may be mounted horizontally or parallel to the base of the chamber 4216, but is preferably angled downwardly such that a portion of the conduit adjacent the side wall of the chamber 4216 but spaced apart therefrom is positioned above the end of the conduit joined to the side wall. Such an embodiment is shown in Figure 45. This promotes flow of the gases down towards the water inside the chamber 4216 in use, directing the gases into contact with the water. This may increase the humidity of gases exiting the chamber 4216. Alternatively, the inlet 4214 may be inclined so as to direct the flow of gases towards the top of the chamber 4216 which may reduce noise created by the flow of gases into and in the chamber 4216. The angle of slope (upwards or downwards) is preferably between 5 degrees and 25 degrees, more preferably between 10 degrees and 20 degrees, and most preferably is approximately 15 degrees.

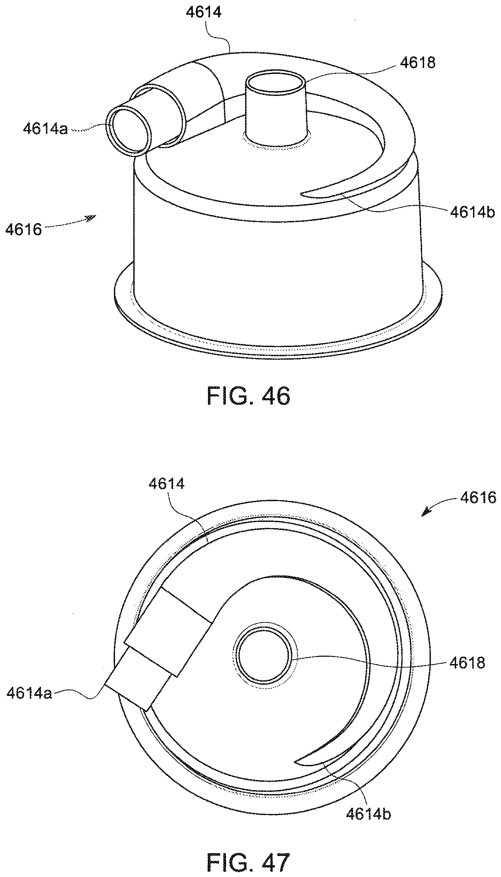

Figs. 46 to 48 show perspective, top and bottom views of a humidification chamber 4616 according to another embodiment. Again, the heat conductive base plate of the chamber has been removed to enable the inside of the chamber to be seen. According to this embodiment, rather than being mounted in or provided to a side wall, the inlet 4614 is provided to the top of the chamber 4616 and is defined by a curved passageway formed in or on the top of the chamber 4616.

As shown in Figs. 46 to 48, the inlet end 4614a of the passageway is configured for attachment to a gases source. For example, as is known in the art, the inlet end 4614 may be provided with an internal or external taper that is configured to mate with and frictionally engage a corresponding connector extending from the gases source. The connector extending from the gases source may extend directly from the gases source or be provided at an end of an elongate flexible conduit or hose, the other end of the conduit or hose being connectable to an outlet of the gases source. The outlet end 4614b comprises an opening that enables gases received at the inlet end 4614a to enter the chamber 4616.

The passageway is arcuate and is preferably configured to be positioned adjacent to but inside of the side wall (when viewed from above), the flow path defined thereby generally matching the shape defined by the inner wall of the chamber 4616, in the embodiment shown, generally circular. Thus the passageway imparts a flow on the gases entering the chamber 4616 that already closes matches the internal contours of the chamber 4616, improving vortex generation and reducing flow scattering.

Further, in the embodiment shown, the top of the passageway is angled towards the base of the chamber 4616 moving from the inlet end 4614a to the outlet end 4614b, and more generally it is shown as tapering (both width and height). The taper more generally serves to increase the velocity of the gases and may provides for a more jet-like flow, or a faster flow, of gases into the chamber 4616.

Further as shown in Figs. 46 to 48, the opening forming the outlet of the passageway may extend along a substantial portion of the base of the passageway (i.e. the part of the passageway closest to the chamber 4616 base. The extent of the opening may be reduced such that the opening is provided along a smaller portion of the passageway. Further, the passageway may extend about a smaller or greater extent of the top of the chamber 4616. As shown, the chamber outlet 4618 is preferably positioned in the top wall of the chamber 4616, generally centrally, but may be otherwise positioned, including offset from the centre of the top and may be provided in the chamber 4616 side wall, preferably an upper portion thereof.

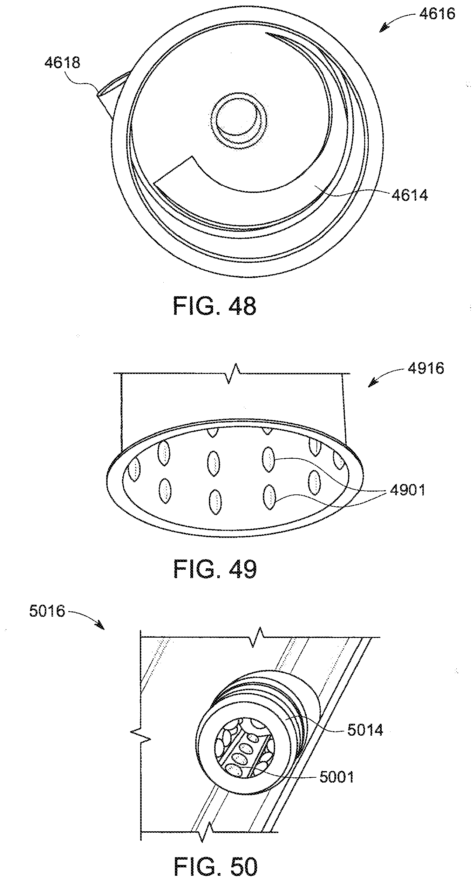

According to some further embodiments, the chamber and/or inlet may comprise one or more flow modifying features. For example, as shown in Fig. 49, the internal wall of the chamber 4916 may be provided with one or more dimples or projections 4901. Additionally or alternatively, as shown in Fig. 50, the inlet 5014 of the chamber 5016 may comprise one or more dimples or projections 5001 on an inner surface thereof. While only parts of the chambers are shown in Figs. 49 and 50, such features 4901, 5001 may be applied to any of the embodiments described herein. The projections may be substituted or complemented with recesses. Such features increase turbulence and/or mixing of gases within the chamber and also boosts the volume of gases that contact water inside the chamber. Thus heat transfer and/or mass transfer and moisture uptake by the gases may be increased. Further the turbulence may increase gas flow path, thereby increasing gas residence time, further improving moisture uptake by the gases.

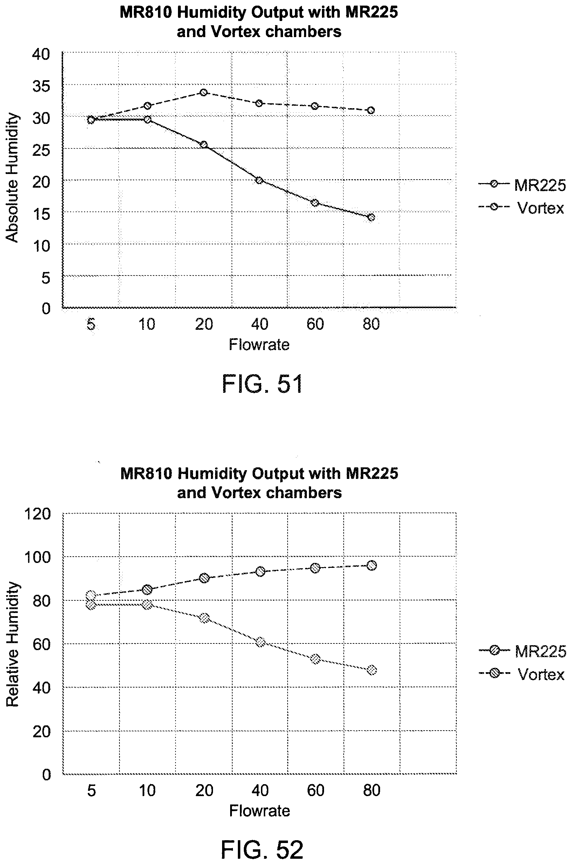

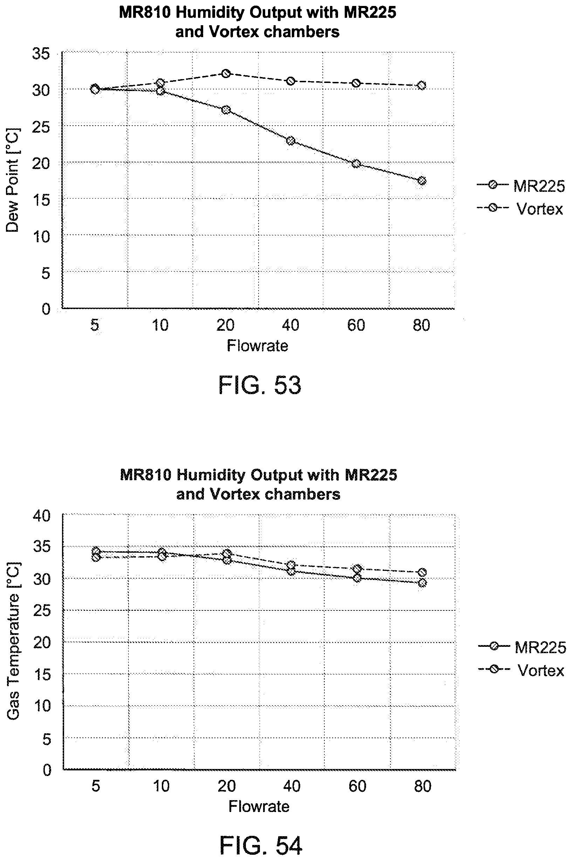

As can be seen from Figures 51 to 54, these show test results of tests carried our using a prior Fisher & Paykel Healthcare Limited MR810 heater base with a prior MR225 humidification chamber and a humidification chamber in accordance with this disclosure. The same MR810 heater base was used in order to provide consistency for both tests. As the graphs show, the humidification chamber in accordance with this disclosure provides improved absolute and relative humidities, improved dew point and improved gas temperature. In each case the improvement increase relatively for higher flow rates.

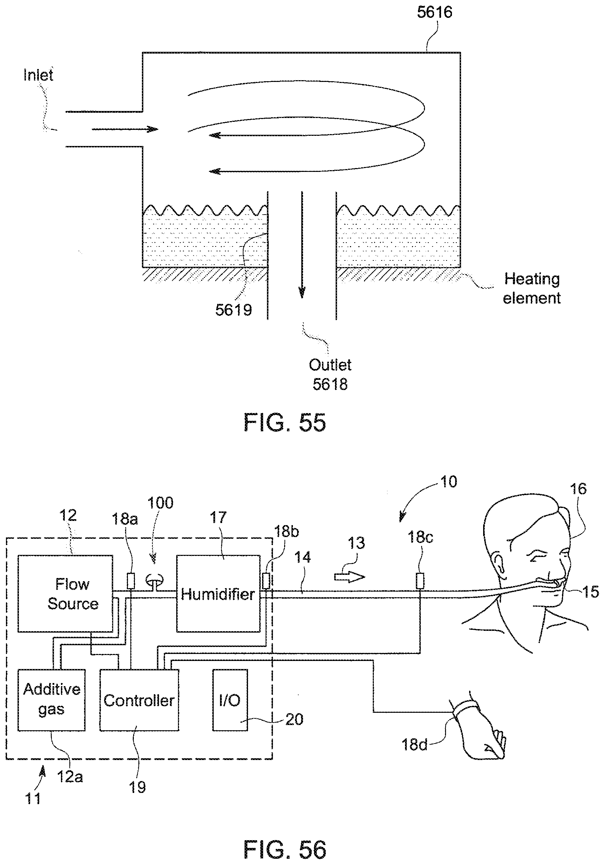

With reference to Figure 55, a further humidification chamber 5616 is shown, which works in a similar manner to the chambers of Figures 2 and 38. In this embodiment, the outlet 5618 is in the base, adjacent the heater plate (although the heater may alternatively or additionally extend up the side wall of the chamber 5616). The remaining chamber features, and the principles of operation, are similar to chamber 216 for example. The outlet 5618 includes an upwardly extending outlet wall 5619 that project into the interior space of the chamber, and which project up from the interior surface of the base, to prevent or minimise water splashing back through the outlet 5618. The outlet 5618 with the upwardly extending wall 5619 may also provide a central structure for the water to rotate around thereby moving the water and improving heat transfer and/or mass transfer through the water. The moving water also improves humidification of the gases due to an improved heat and mass transfer coefficient.

**Further explanatory comments and summary of possible advantages**

We provide below some further explanation of one or more of the possible operating principles, and one or more of the possible advantages, provided by humidification chambers in accordance with this disclosure.

Maximizing the path length of the gas flow within the chamber can be achieved by positioning the inlet and outlet on the chamber such that a vortex flow is created within the humidification chamber. The vortex allows for repeated rotations of gas within the chamber increasing the distance travelled by the gas. The gas is therefore in contact with wetted surfaces over a greater distance, increasing the opportunity for humidification.

Increasing the velocity of the gas within the chamber can be achieved by providing the inlet at a position on the side wall of the humidification chamber such that the direction of the gases flow is substantially tangential to the side wall and the outlet at a central location on the upper wall. In addition, increasing the gases velocity over a liquid also increases heat and mass transfer and therefore moisture pick-up. The continuous injection of a flow at the inlet in a direction substantially tangential to the side wall causes the gases and a vortex to accelerate to a velocity to near the injection velocity. The entire water surface area is therefore exposed to relatively fast moving gases which increases the humidification efficiency. Increasing the gases velocity increases the Reynolds number and turbulence of the gases flow in the chamber. Increasing the turbulence increases mixing and scrubs the boundary layer above the surface of the liquid water, thereby increasing the humidity gradient. Increasing the gases velocity further causes the distance between adjacent winds of the spiral/vortex to reduce, therefore increasing the gas flow path length.

The vortex or spiraling flow increases moisture pick-up via surface area by increasing the efficient use of the surface area available for heat transfer and/or mass transfer and/or increasing the actual surface available for heat transfer and/or mass transfer. Increasing the actual surface area can be achieved by causing ripples in the water surface, the rotating water being thrown up the chamber side walls and, at high flow rates, disrupting the liquid surface tension to cause the water to splash and expose the additional liquid water surface area of numerous droplets. The vortex causes the entirety of, or at least more of, the gases within the chamber to circulate and there are no regions of, or at least less regions of, stagnant gases flow. Therefore, the entire surface area, or at least more of the surface area, of the liquid is being exposed to gases moving over it and picking-up moisture.

Conservation of mass in the sealed chamber requires that what goes into the chamber must come out. The average residence time of the gas within the chamber is therefore only dependent on the volume of the chamber and the input flowrate (and to a lesser extent the volume expansion of the gas due to heating and the additional gas volume of evaporated water). The average residence time of chambers of equal volume and input flowrate should therefore be comparable. The residence time of individual pockets of gas can however differ and can be a function of chamber design. For example a chamber with regions of stagnant flow and fast moving flow could result in some gas spending a relatively long time in the chamber and some gas a relatively short time. The standard deviation in residence time is therefore relatively large. Neither the stagnant gas, which is somewhat isolated from the flow stream, nor the gas that spends the shortest time in the chamber, contribute much to the humidification of the main flow stream. The vortex causes the entirety of, or at least an increased amount of, the gases within the chamber to circulate such that there are no regions of stagnant gases flow and all the gas is rotating at substantially the same speed. The vortex can therefore create more efficient humidification.

The vortex can encourage the humidified gases to exit the humidification chamber before the non-humidified gases. Furthermore, warm gases are less dense (i.e. lighter) than cool gases. When the gases are rotating, the centripetal acceleration causes a centripetal force on the gases. This centripetal force (FC) is proportional to the gases density ($\rho$), velocity ($v$) and radius of curvature ($r$) and is given by the following equation:

$$F_C = (\rho v^2)/r$$

Therefore, the dry and denser gases experience a greater force in relation to the humidified and lighter gases, thus the dry and denser gas are pushed towards the outside of the chamber and away from the central exit outlet. The warmed and humidified gases exit preferentially from the central exit outlet.

Improving the heat transfer and/or mass transfer can be achieved by the movement of water. The rotation of the gases induces a rotation of the water due to viscous shear. The movement of the water over the chamber improves the efficiency of the heat transfer and/or mass transfer from the heater plate into the water itself. This is advantageous because a lower heater plate temperature will be necessary for a desired heat input.

Further, improving the performance over a wide range of flow rates can be achieved by using a vortex gases flow within the humidification chamber. Humidification chambers typically suffer performance issues at higher flow rates as these tend to reduce the residence time of gases inside the humidification chamber. The vortex humidification chamber provides a more reliable performance across a wide range of flow rates. This is exemplified in the test results of Figures 51 to 54.

The humidification chambers described herein can also be used in respiratory humidification applications. The humidification chamber may be used in invasive or non invasive ventilation or CPAP or nasal high flow delivery. The humidification chambers described herein can provide similar benefits of improved humidification efficiency that can result in lower power being used and a more stable i.e. flat humidity response over a wide range of flow ranges.

The humidification chambers can be used in any humidification system for medical or respiratory therapy. We set out below three example humidification systems in which a humidification chamber in accordance with any of the above disclosure, or aspects of the disclosure, may be used. References in the following description to 'chamber' are to be taken as references to any one of the humidification chambers disclosed above.

**Anesthesia Humidification System**

A humidification chamber according to embodiments described herein is particularly adapted for use in respiratory systems such as CPAP or high flow respiratory gas systems, for example a high flow system for use in anaesthesia procedures. Such a system is shown schematically in Figure 56. Respiratory systems in which the chamber may be particularly useful are CPAP, BiPAP, high flow therapy, varying high flow therapy, low flow air, low flow O2 delivery, bubble CPAP, apnoeic high flow (i.e. high flow to anesthetized patients), invasive ventilation and non-invasive ventilation. Further, a chamber as described herein may be useful in systems other than respiratory systems.

Unless the context suggests otherwise, a flow source provides a flow of gases at a set flow rate. A set flow rate may be a constant flow rate, variable flow rate or may be an oscillating flow rate, for example a sinusoidal flow rate or a flow rate with a step or square wave profile. Unless the context suggests otherwise a pressure source provides a flow of gases at a set pressure. The set pressure may be a constant pressure, variable pressure or may be an oscillating pressure, for example a sinusoidal pressure or a pressure with a step or square wave profile.

'High flow therapy' as used in this disclosure may refer to delivery of gases to a patient at a flow rate of greater than or equal to about 5 or 10 litres per minute (5 or 10 LPM or L/min).

In some configurations, 'high flow therapy' may refer to the delivery of gases to a patient at a flow rate of about 5 or 10 LPM to about 150 LPM, or about 15 LPM to about 95 LPM, or about 20 LPM to about 90 LPM, or about 25 LPM to about 85 LPM, or about 30 LPM to about 80 LPM, or about 35 LPM to about 75 LPM, or about 40 LPM to about 70 LPM, or about 45 LPM to about 65 LPM, or about 50 LPM to about 60 LPM. For example, according to those various embodiments and configurations described herein, a flow rate of gases supplied or provided to an interface via a system or from a flow source, may comprise, but is not limited to, flows of at least about 5, 10, 20, 30, 40, 50, 60, 70, 80, 90, 100, 110, 120, 130, 140, 150 LPM, or more, and useful ranges may be selected to be any of these values (for example, about 20 LPM to about 90 LPM, about 40 LPM to about 70 LPM, about 40 LPM to about 80 LPM, about 50 LPM to about 80 LPM, about 60 LPM to about 80 LPM, about 70 LPM to about 100 LPM, about 70 LPM to about 80 LPM).

The gas delivered will be chosen depending on the intended use of the therapy. Gases delivered may comprise a percentage of oxygen. In some configurations, the percentage of oxygen in the gases delivered may be about 15% to about 100%, 20% to about 100%, or about 30% to about 100%, or about 40% to about 100%, or about 50% to about 100%, or about 60% to about 100%, or about 70% to about 100%, or about 80% to about 100%, or about 90% to about 100%, or about 100%, or 100%.

In some embodiments, gases delivered may comprise a percentage of carbon dioxide. In some configurations, the percentage of carbon dioxide in the gases delivered may be about 20% to about 100%, or about 30% to about 100%, or about 40% to about 100%, or about 50% to about 100%, or about 60% to about 100%, or about 70% to about 100%, or about 80% to about 100%, or about 90% to about 100%, or about 100%, or 100%.

High flow therapy has been found effective in meeting or exceeding the patient's normal real inspiratory demand, to increase oxygenation of the patient and/or reduce the work of breathing. Additionally, high flow therapy may generate a flushing effect in the nasopharynx such that the anatomical dead space of the upper airways is flushed by the high incoming gas flows. This creates a reservoir of fresh gas available of each and every breath, while minimising re-breathing of carbon dioxide, nitrogen, etc.

By example, a high flow respiratory system 10 is described with reference to Figure 56. High flow therapy may be used as a means to promote gas exchange and/or respiratory support through the delivery of oxygen and/or other gases, and through the removal of $CO_2$ from the patient's airways. High flow therapy may be particularly useful prior to, during or after a medical procedure.

When used prior to a medical procedure, high gas flow can pre-load the patient with oxygen so that their blood oxygen saturation level and volume of oxygen in the lungs is higher to provide an oxygen buffer while the patient is in an apnoeic phase during the medical procedure.

A continuous supply of oxygen is essential to sustain healthy respiratory function during medical procedures (such as during anaesthesia) where respiratory function might be compromised (e.g. diminishes or stops). When this supply is compromised, hypoxia and/or hypercapnia can occur. During medical procedures such as anaesthesia and/or general anaesthesia where the patient is unconscious, the patient is monitored to detect when this happens. If oxygen supply and/or $CO_2$ removal is compromised, the clinician stops the medical procedure and facilitates oxygen supply and/or $CO_2$ removal. This can be achieved for example by manually ventilating the patient through an anaesthetic bag and mask, or by providing a high flow of gases to the patient's airway using a high flow therapy system.

Further advantages of high gas flow can include that the high gas flow increases pressure in the airways of the patient, thereby providing pressure support that opens airways, the trachea, lungs/alveolar and bronchioles. The opening of these structures enhances oxygenation, and to some extent assists in removal of $CO_2$.

The increased pressure can also keep structures such as the larynx from blocking the view of the vocal chords during intubation. When humidified, the high gas flow can also prevent airways from drying out, mitigating mucociliary damage, and reducing risk of laryngospasms and risks associated with airway drying such as nose bleeding, aspiration (as a result of nose bleeding), and airway obstruction, swelling and bleeding. Another advantage of high gas flow is that the flow can clear smoke created during surgery in the air passages. For example, smoke can be created by lasers and/or cauterizing devices.

A pressure relief or regulating device is particularly desirable for use in a respiratory system such as a high flow system comprising an unsealed patient interface, to provide an upper pressure limit for the system. Most importantly, the upper pressure limit may be configured to provide a patient safety limit, or may be configured to prevent damage to tubes, fluid connections, or other components. A pressure relief or regulating device may be used in a CPAP (continuous positive airway pressure), BiPAP (bilevel positive airway pressure) and/or Bubble CPAP systems to regulate the pressure provided to the patient.

With reference to Figure 56, the system/apparatus 10 may comprise an integrated or separate component based arrangement, generally shown in the dotted box 11 in Figure 1A. In some configurations the system 10 could comprise a modular arrangement of components. Hereinafter the system/apparatus 10 will be referred to as system, but this should not be considered limiting. The system 10 may include a flow source 12, such as an in-wall source of oxygen, an oxygen tank, a blower, a flow therapy apparatus, or any other source of oxygen or other gas. The system 10 may also comprise an additive gas source 12a, comprising one or more other gases that can be combined with the flow source 12. The flow source 12 can provide a pressurised high gas flow 13 that can be delivered to a patient 16 via a delivery conduit 14, and patient interface 15 (such as a nasal cannula). A controller 19 controls the flow source 12 and additive gas source 12a through valves or the like to control flow and other characteristics such as any one or more of pressure, composition, concentration, volume of the high flow gas 13. A humidifier 17 is also provided, which can humidify the gas under control of the controller and control the temperature of the gas. The humidifier 17 comprises a humidification chamber as disclosed above. One or more sensors 18a, 18b, 18c, 18d, such as flow, oxygen, pressure, humidity, temperature or other sensors can be placed throughout the system and/or at, on or near the patient 16. The sensors can include a pulse oximeter 18d on the patient for determining the oxygen concentration in the blood.

The controller 19 may be coupled to the flow source 12, the additive gas source 12a, humidifier 17 and sensors 18a-18d. The controller 19 can operate the flow source to provide the delivered flow of gas. It can control the flow, pressure, composition (where more than one gas is being provided), volume and/or other parameters of gas provided by the flow source based on feedback from sensors. The controller 19 can also control any other suitable parameters of the flow source to meet oxygenation requirements. The controller 19 can also control the humidifier 17 based on feedback from the sensors 18a-18d. Using input from the sensors, the controller can determine oxygenation requirements and control parameters of the flow source 12 and/or humidifier 17 as required. An input/output (I/O) interface 20 (such as a display and/or input device) is provided. The input device is for receiving information from a user (e.g. clinician or patient) that can be used for determining oxygenation requirements. In some embodiments, the system may be without a controller and/or I/O interface. A medical professional such as a nurse or technician may provide the necessary control function.

The pressure may also be controlled. As noted above, the high gas flow (optionally humidified) can be delivered to the patient 16 via a delivery conduit 14 and the patient interface 15 or 'interface', such as a cannula, mask, nasal interface, oral device or combination thereof. In some embodiments, the high gas flow (optionally humidified) can be delivered to the patient 16 for surgical uses, e.g. surgical insufflation. In these embodiments, the 'interface' could be a surgical cannula, trocar, or other suitable interface. The patient interface can be substantially sealed, partially sealed or substantially unsealed. A nasal interface as used herein is a device such as a cannula, a nasal mask, nasal pillows, or other type of nasal device or combinations thereof. A nasal interface can also be used in combination with a mask or oral device (such as a tube inserted into the mouth) and/or a mask or oral device (such as a tube inserted into the mouth) that can be detached and/or attached to the nasal interface. A nasal cannula is a nasal interface that includes one or more prongs that are configured to be inserted into a patient's nasal passages. A mask refers to an interface that covers a patient's nasal passages and/or mouth and can also include devices in which portions of the mask that cover the patient's mouth are removable, or other patient interfaces such as laryngeal mask airway or endotracheal tube. A mask also refers to a nasal interface that includes nasal pillows that create a substantial seal with the patient's nostrils. The controller controls the system to provide the required oxygenation.

**High Flow Humidification System**

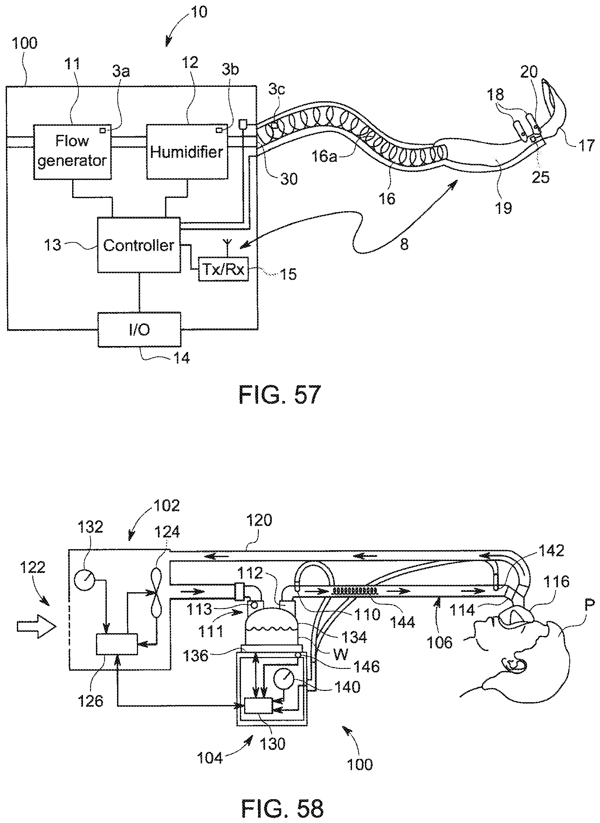

With reference to Figure 57, a humidification chamber according to embodiments described herein is particularly adapted for use in a breathing assistance or respiratory therapy apparatus 10 for delivering a flow of gas (which may contain one or more gases) to a patient. The apparatus 10 could, for example, be a CPAP apparatus or a high flow apparatus. An exemplary CPAP apparatus is described in WO 2011/056080.

In general terms, the apparatus 10 comprises a main housing 100 that contains a flow generator 11 in the form of a motor/impeller arrangement, a humidifier 12, a controller 13, and a user I/O interface 14 (comprising, for example, a display and input device(s) such as button(s), a touch screen, or the like). The humidifier 12 comprises a humidification chamber as disclosed above. The controller 13 is configured or programmed to control the components of the apparatus, including: operating the flow generator 11 to create a flow of gas (gas flow) for delivery to a patient, operating the humidifier 12 to humidify and/or heat the generated gas flow, receive user input from the user interface 14 for reconfiguration and/or user-defined operation of the apparatus 10, and output information (for example on the display) to the user. The user could be a patient, healthcare professional, or anyone else interested in using the apparatus.

An alternative form breathing assistance apparatus may be a standalone humidifier apparatus comprising a main housing and a humidifier 12. An exemplary standalone humidifier apparatus is described in WO 2015/038013.

A patient breathing conduit 16 is connected to a gas flow output or patient outlet port 30 in the housing 100 of the breathing assistance apparatus 10, and is connected to a patient interface 17 such as a nasal cannula with a manifold 19 and nasal prongs 18. Additionally, or alternatively, the patient breathing conduit 16 could be connected to a face mask. Additionally, or alternatively, the patient breathing conduit could be connected to a nasal pillows mask, and/or a nasal mask, and/or a tracheostomy interface, or any other suitable type of patient interface. The gas flow, which may be humidified, that is generated by the breathing assistance apparatus 10 is delivered to the patient via the patient breathing conduit 16 through the patient interface 17. The patient breathing conduit 16 can have a heater wire 16a to heat gas flow passing through to the patient. The heater wire 16a is under the control of the controller 13. The patient breathing conduit 16 and/or patient interface 17 can be considered part of the breathing assistance apparatus 10, or alternatively peripheral to it. The breathing assistance apparatus 10, breathing conduit 16, and patient interface 17 may together form a breathing assistance system or, in some configurations, a flow therapy system.

General operation of an exemplary breathing assistance apparatus 10 will be known to those skilled in the art, and need not be described in detail here. However, in general terms, the controller 13 controls the flow generator 11 to generate a gas flow of the desired flow rate, controls one or more valves to control the mix of air and oxygen or other alternative gas, and controls the humidifier 12 to humidify the gas flow and/or heat the gas flow to an appropriate level. The gas flow is directed out through the patient breathing conduit 16 and patient interface 17 to the patient. The controller 13 can also control a heating element in the humidifier 12 and/or the heating element 16a in the patient breathing conduit 16 to humidify and/or heat the gas to a desired temperature that achieves a desired level of therapy and/or comfort for the patient. The controller 13 can be programmed with, or can determine, a suitable target temperature of the gas flow.

Operation sensors 3a, 3b, 3c, 20, and 25, such as flow, temperature, humidity, and/or pressure sensors, can be placed in various locations in the breathing assistance apparatus 10 and/or the patient breathing conduit 16 and/or patient interface 17. Output from the sensors can be received by the controller 13, to assist it to operate the breathing assistance apparatus 10 in a manner that provides optimal therapy. In some configurations, providing optimal therapy includes meeting a patient's inspiratory demand. The apparatus 10 may have a transmitter and/or receiver 15 to enable the controller 13 to receive signals 8 from the sensors and/or to control the various components of the breathing assistance apparatus 10, including but not limited to the flow generator 11, humidifier 12, and heater wire 16a, or accessories or peripherals associated with the breathing assistance apparatus 10. Additionally, or alternatively, the transmitter and/or receiver 15 may deliver data to a remote server or enable remote control of the apparatus 10.

The breathing assistance apparatus 10 may be any suitable type of apparatus, but in some configurations may deliver a high gas flow or high flow therapy (of e.g. air, oxygen, other gas mixture, or some combination thereof) to a patient to assist with breathing and/or treat breathing disorders. In some configurations, the gas is or comprises oxygen. In some configurations, the gas comprises a blend of oxygen and ambient air. As used herein, 'high flow' therapy refers to administration of gas to the airways of a patient at a relatively high flow rate that meets or exceeds the peak inspiratory demand of the patient. The flow rates used to achieve 'high flow' may be any of the flow rates listed below. For example, in some configurations, for an adult patient 'high flow therapy' may refer to the delivery of gases to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult patient, a neonatal, infant, or child patient, may deliver gases to the patient at a flow rate of between about 1 LPM and about 100 LPM, or at a flow rate in any of the sub-ranges outlined above. Gases delivered may comprise a percentage of oxygen. In some configurations, the percentage of oxygen in the gases delivered may be between about 20% and about 100%, or between about 30% and about 100%, or between about 40% and about 100%, or between about 50% and about 100%, or between about 60% and about 100%, or between about 70% and about 100%, or between about 80% and about 100%, or between about 90% and about 100%, or about 100%, or 100%.

During high flow therapy the delivered gas flow will generally meet or exceed the patient's inspiratory demand, which may increase oxygenation of the patient and/or reduce the work of breathing. Additionally, high flow therapy may generate a flushing effect in the nasopharynx such that the anatomical dead space of the upper airways is flushed by the high incoming gas flows. This creates a reservoir of fresh gas available for each and every breath, while minimising re-breathing of carbon dioxide, nitrogen, etc.

The patient interface 17 may be a non-sealing interface to prevent barotrauma (e.g. tissue damage to the lungs or other organs of the respiratory system due to difference in pressure relative to the atmosphere). The patient interface may be a nasal cannula with a manifold and nasal prongs, and/or a face mask, and/or a nasal pillows mask, and/or a nasal mask, and/or a tracheostomy interface, or any other suitable type of patient interface.

**Respiratory Humidification System**

With reference to Figure 58, a humidification chamber according to embodiments described herein is particularly adapted for use in a respiratory assistance system for delivery of heated and humidified gases to a patient can include a patient interface configured to deliver a flow of respiratory gases received from a gases source, an inspiratory conduit configured to be in fluid communication with the patient interface and the gases source via a humidifier. The humidifier can include a humidification chamber, in accordance with the above disclosure, having at least one wall that defines the chamber such that the chamber can hold a liquid, a chamber inlet, a chamber outlet, and a gases flow path between the chamber inlet and the chamber outlet. The chamber inlet can be configured to be in fluid communication with the gases source and the chamber outlet can be configured to be in fluid communication with the inspiratory conduit. The humidification chamber can hold a volume of liquid (such as water). The humidifier can include a heater plate configured to heat the volume of liquid and the flow of respiratory gases in the gases flow path within the humidification chamber so as to heat and humidify the flow of respiratory gases. The humidifier can also include a controller having one or more hardware processors configured to control the amount of power delivered to the heater plate.

The humidifier examples disclosed herein can include a controller configured to change the humidification chamber outlet temperature set point as a function of chamber inlet temperature. For example, the controller can be configured to detect inlet gas temperature. As chamber inlet temperature increases, the controller can decrease the desired humidity level at the outlet to a lower level (such as a lower therapeutic level), allowing and accounting for additional humidity that may be added in the case of a room air entraining ventilator being the gas source that is connected to the humidifier. The controller can decrease the desired humidity level by optionally changing a heater plate power set point or a heater plate temperature set point. These two parameters may be used in addition or in alternative to a chamber outlet set point. The controller may be configured to, if the inlet temperature exceeds a threshold, bound or cap a chamber outlet set point such that the amount of humidity generated by the humidifier is capped to account for the increased humidity in the ambient air. Additionally or alternatively, the power provided to the heater plate may be capped or bound if the inlet gases temperature exceeds a threshold. The controller may also cap or bound the heater plate temperature set point if the inlet gases temperature exceeds a threshold. This process enables the humidifier to maintain and/or deliver a therapeutic level of humidity while reducing condensation forming in the inspiratory tube and/or patient interface as a result of additional humidity in the incoming gas. Thus, the systems and methods described herein can account for different incoming humidity levels in a respiratory assistance system and improve patient comfort by reducing rain out when the incoming gas has a humidity greater than a dry gas.

The humidifier and humidifier controller disclosed herein may be configured to control a humidifier to operate in two modes, a first mode being a dry inlet gases mode and a second mode being a humidified inlet gases mode. The mode of operation being controlled based on the temperature of the inlet gases. If the temperature of the inlet gases, that is, the inlet temperature is below a threshold the humidifier functions in a first mode. If the temperature of the inlet gases exceeds a threshold, (that is, the inlet temperature exceeds a threshold) the controller operates in a second mode. The second mode reduces the humidity output of the humidifier. This is achieved by capping or bounding the chamber outlet temperature set point in order to reduce the amount of humidity generated by the humidifier. Alternatively, the controller may cap or bound the heater plate set point temperature or heater plate power in order to reduce or cap the humidity generated in the second mode as compared to the first mode. The second mode compensates for humidity in the inlet gases e.g. ambient air.

The humidifier and methods of use described herein can be used to provide non-invasive therapies and/or invasive therapies. The humidifier can be operated in invasive mode, non-invasive mode, high flow mode, or other modes. The humidifier can operate with various patient interfaces such as, for example, an endotracheal tube (ET tube), full face mask, nasal mask, nasal cannula, nasal pillows, sealed prongs, or any other interface. Other desired humidity levels may be possible and other types of therapy systems may be used. The chamber outlet temperature set point can be adjusted according to the therapies provided and the desired humidity levels.

FIG. 58 shows a schematic of an example respiratory assistance system 100. As illustrated, the respiratory assistance system 100 includes a humidifier 104, a gas source 102, a patient interface 116, and an inspiratory conduit 106 configured to transport respiratory gases from the humidifier 104 to the patient interface 116. The gas source 102 and the humidifier 104 may be in separate housings, or optionally may be co-located, within the same housing, and/or included in a single apparatus. The humidifier 104 comprises a humidification chamber as disclosed above. The respiratory assistance system 100 includes an optional expiratory conduit 120 configured to transport gases from the patient interface 116 to the gas source 102 and an optional wye-piece 114 configured to connect the inspiratory conduit 106 and the expiratory conduit 120 to the patient interface 116. The respiratory assistance system 100 may not include the expiratory conduit 120 or may include an exhalation port. The operating parameters of the respiratory assistance system 100 may need to be adjusted depending on whether an expiratory conduit or an exhalation port is included.

As illustrated, the gas source 102 includes a ventilator 124, which may include a blower or alternatively a turbine. The gas source 102 may also include other mechanisms to deliver or push a flow of respiratory gases to the humidifier 104, such as a valve arrangement or a pump. The gas source 102 is an example room or ambient air entraining ventilator. The gas source 102 may include an inlet 122 through which ambient air is drawn into the gas source 102, for example, by the ventilator 124. The gas source 102 may optionally include a controller 126 configured to control the operation of the ventilator 124. The gas source 102 may optionally include a user interface 132 that can provide information regarding user input to the controller 126. The controller 126 can control the operation of the ventilator 124 based on information provided by the user interface 132 and/or based on other information, for example but not limited to, feedback from the ventilator 124, such as from a sensor associated with the ventilator 124. Instead of drawing ambient air, the inlet 122 can be connected to a supply of dry gas, for example, a gas canister or tank. These type of ventilators can be referred to non-entraining ventilators and may be controlled by one or more valves such as proportional valves. The valve or valves may be controlled by a controller, such as the controller 126.

The humidifier 104 may include a humidification chamber 134 and a heater plate 136. The humidification chamber 134 may be configured to hold a volume of water W or other suitable liquid, and comprises features in accordance with those of the humidification chambers disclosed above. The heater plate 136 may be configured to heat the volume of water W and respiratory gases within the humidification chamber 134, which may increase the temperature of the respiratory gases and may create vapor from the volume of water W that is taken up by the respiratory gases. The humidification chamber 134 may include a chamber inlet 111 and a chamber outlet 112. The inspiratory conduit 106 may be configured to be connected to the chamber outlet 112, such that heated and humidified respiratory gases may be transported by the inspiratory conduit 106 from the humidification chamber 134 to the patient interface 116 and then delivered to a patient P. Gases exhaled by the patient P into the patient interface 116 may optionally be returned by the expiratory conduit 120 to the gas source 102. The respiratory assistance system 100 may not include the expiratory conduit 120 and thus gases exhaled by the patient P into the patient interface 116 may be vented to the atmosphere, such as directly, or optionally through an exhalation port.

The humidifier 104 may include a controller 130 that can control, for example but not limited to, the operation of the heater plate 136. When the humidifier 104 and the gas source 102 form an integrated device, the controller 126, 130 may be the same hardware processor or separate processors. The humidifier 104 may also include a user interface 140 for providing and/or receiving information regarding user input to the controller 130. The humidifier 104 may further include an inlet temperature sensor 113. The inlet temperature sensor 113 may be configured to detect the temperature of gases entering the humidifier. The inlet temperature sensor 113 may measure a characteristic of the ambient air near the location of the inlet temperature sensor 113, such as a temperature of the ambient air. The inlet temperature sensor 113 can also be a temperature sensor located at or near the chamber inlet 111. The temperature sensor at the chamber inlet 111 can optionally measure both temperature and flow rate of the air coming in from the gas source 102. This measurement can provide an indication of ambient conditions. Additionally and/or alternatively, the respiratory assistance system 100 may include more than one sensor located at or near the chamber inlet 111. The inlet sensors can include a temperature sensor and a separate flow sensor. The one or more inlet sensors can be located at any location from the gas source 102 to the humidification chamber 134. The one or more outlet sensors 110 and the one or more inlet sensors may be integrated with the humidification chamber 134. The controller 130 may receive information regarding a characteristic of the ambient air near the location of the inlet temperature sensor 113 from the inlet temperature sensor 113. The controller 130 may be configured to control the operation of the heater plate 136 based on information provided by the user interface 140, based on information provided by the inlet temperature sensor 113, and/or based on other information, for example but not limited to, feedback from the heater plate 136, such as from a temperature sensor 146 located at or near the heater plate 136. The controller 130 may be configured to determine an amount of power, or a power duty cycle, to provide to the heater plate 136 such that the heater plate 136 delivers a desired amount of heat to respiratory gases and the volume of water W within the humidification chamber 134.

The respiratory assistance system 100 may include one or more outlet sensors 110 that are associated with the chamber outlet location 112. The one or more outlet sensors 110 may also be located at or near the chamber outlet 112. The outlet sensors 110 can include two sensors: a temperature sensor and a flow sensor. The temperature sensor can be a thermistor (such as a heated thermistor). The thermistor can also be used as a flow sensor. Accordingly, there may be a single sensor 110 at or near the chamber outlet 112. Other types of temperature sensors and flow sensors that can work in a respiratory assistance system 100 may also be used. The outlet sensor(s) 110 may be located at the chamber outlet 112, at the inspiratory conduit 106 near the connection between the chamber outlet 112 and the inspiratory conduit 106, or at another suitable location downstream of the humidification chamber 134. The controller 130 may receive information from the outlet sensor(s) 110 regarding a characteristic of respiratory gases flowing past the location of the outlet sensor 110. The controller 130 may be configured to control the operation of the heater plate 136 based on information provided by the outlet sensor(s) 110, instead of or in addition to other sources of information as previously described. An outlet sensor 110 may be integrated into the heater base or may be disposed on a cartridge that is removably attachable to a vertical portion of a heater base. The sensors may be insertable into the inlet port and outlet port as the chamber 134 is positioned in an operative position on the heater base. The chamber inlet and outlet may include openings that correspond to the inlet temperature sensor 113 and outlet sensor 110 to receive the sensors. The sensor openings in the chamber may include polymer covers that are configured to cover the sensor tip as the sensors are inserted into the gases path such that the sensors do not need to sterilized, since the sensors are not actually in contact with the gases.

Respiratory gases flowing through the inspiratory conduit 106 may lose heat through the walls of the inspiratory conduit 106, which may reduce the temperature of the respiratory gases and may cause condensation to form within the inspiratory conduit 106. The inspiratory conduit 106 may include a conduit heater 144 configured to heat the inspiratory conduit 106 to reduce or prevent this loss of heat. The controller 130 may be configured to control the operation of the conduit heater 144 based on one or several sources of information as previously described. In particular, the controller 130 may be configured to determine an amount of power, or a power duty cycle, to provide to the conduit heater 144 such that the conduit heater 144 delivers a desired amount of heat to the inspiratory conduit 106. The conduit heater may be disposed into the wall of the conduit or may be disposed within the lumen of the conduit.

The respiratory assistance system 100 may include one or more conduit sensors 142 located within the inspiratory conduit 106. The conduit sensor(s) 142 may be located at the inspiratory conduit 106 near the connection between the inspiratory conduit 106 and the wye-piece 114, at the connection between the inspiratory conduit 106 and the patient interface 116 if the inspiratory conduit 106 is connected directly to the patient interface 116, or at the wye-piece 114 or the patient interface 116. The conduit sensor(s) 142 may measure a characteristic of respiratory gases flowing past the location of the conduit sensor 142, such as a temperature of the respiratory gases. The conduit sensor 142 can include a temperature sensor. The conduit sensor 142 can also include a separate flow sensor. The conduit sensor 142 can include an integral flow and temperature sensor that is capable of measuring both the temperature and flow rate such as described herein. The controller 130 may receive information regarding a characteristic of respiratory gases flowing past the location of the conduit sensor 142 from the conduit sensor 142. The controller 130 may determine the flow rate of respiratory gases flowing past the conduit sensor 142. The controller 130 may be configured to control the operation of the conduit heater 144, and/or the operation of the heater plate 136, based on information received from the conduit sensor 142, instead of or in addition to other sources of information as previously described. The conduit sensor may be integrated into the conduit and extend into the gases pathway defined by the conduit. Further, the conduit sensor's wires may be integrated into the wall of the conduit or extend along the conduit.

Respiratory gases may also lose heat through the walls of the patient interface 116, the wye-piece 114, and/or any other respiratory system component that may connect the patient interface 116 to the inspiratory conduit 106. One or more of the patient interface 116, the wye-piece 114, and any other respiratory system component that may connect the patient interface 116 to the inspiratory conduit 106 may include an associated heater and/or an associated sensor. The controller 130 may receive information from such an associated sensor regarding a characteristic of respiratory gases flowing past the location of the sensor. The controller 130 may use information received from such an associated sensor to control the operation of the respective associated heater.

One or more of the patient interface 116, the wye-piece 114, and any other respiratory system component that may connect the patient interface 116 to the inspiratory conduit 106 may not include an associated heater and/or an associated sensor. The controller 130 may use an estimate of the heat lost by respiratory gases flowing through unheated respiratory system components to control other heaters associated with the humidifier 104, such as the heater plate 136 and/or the conduit heater 144. The controller 130 may calculate such a heat loss estimate for unheated respiratory system components based on other received information, such as, but not limited to, information received from the outlet sensor 110, the conduit sensor 142, the inlet temperature sensor 113, and/or the user interface 140, and/or based on information retrieved from a data storage device, which may be located in the controller. The data received from the sensors described herein can also be stored in the data storage device.

The humidifier 104 may be used in the respiratory assistance system 100 to deliver heated and humidified respiratory gases to the patient P for multiple types of respiratory therapies, including but not limited to invasive ventilation therapy, non-invasive ventilation therapy, high flow therapy, BiPaP therapy, Continuous Positive Airway Pressure therapy, or other respiratory assistance therapy. The humidity conditions of the respiratory gases provided to the humidifier 104 by the gas source 102 may vary. For example, the type of the gas source 102 used in the respiratory assistance system 100 may depend on the type of respiratory therapy, respiratory system configurations, location of use (such as home or hospital), or availability of different gas supplies. Gases from different supplies may have different characteristics, including temperature and humidity. Ambient air, in particular, ambient air in tropical weather and/or during summer time can have a higher humidity than gas obtained from a compressed gas tank or bottle. It may be beneficial to adjust the operating parameters of the respiratory assistance system 100 using a control system such that the patient receives comfortable care, for example, with reduced and/or minimized rain out in the inspiratory tube and/or patient interface while still receiving adequately humidified gases in spite of different supply gas characteristics. The control system may be able to automatically adjust operating parameters based on an inference of whether the supply gas is dry or ambient. The operating parameters may include certain temperature set points described below. Additionally or alternatively, the operating parameters may be a dew point, humidity output of the humidifier, or other suitable parameter.

There have been described and illustrated herein several embodiments of a humidification chamber. While particular embodiments have been described, it is not intended that the disclosure be limited thereto, as it is intended that the disclosure be as broad in scope as the art will allow and that the specification be read likewise. Thus, while particular types of inlet configurations/arrangements, outlet configurations/arrangements, heater plate, heating elements, protrusions, number, size, shape, for the humidification chambers have been disclosed, it will be appreciated that any suitable combination of these features may be used to provide a vortex humidification chamber. In addition, while particular types of inlet configurations/arrangements, outlet configurations/arrangements, heater plate, heating elements, protrusions, number, size, shape, for the humidification chambers etc. have been disclosed, it will be understood that other types can be used. It will therefore be appreciated by those skilled in the art that yet other modifications could be made to the disclosed embodiments without deviating from their scope as disclosed or claimed.

**Claims**

1. A humidification chamber for use in a medical humidification system, the humidification chamber comprising:

    a base and a top linked by a side wall to define the chamber;
    a gases inlet configured to receive a gases flow from a gases source; and
    a gases outlet,
    wherein the gases inlet is orientated relative to the side wall of the chamber to introduce the gases flow to the humidification chamber as a gases jet in a direction substantially tangential to the side wall of the humidification chamber, and **characterized in that** the gases inlet comprises a nozzle having a constriction that internally tapers so as to be gradually narrowed along its length, wherein the internal profile is substantially smooth.

2. The humidification chamber of claim 1, wherein the side wall defines a substantially circular chamber, when viewed from above.

3. The humidification chamber of claim 1 or 2, wherein the gases outlet is disposed on the top of the humidification chamber.

4. The humidification chamber of any one of the preceding claims, wherein the gases inlet comprises or is configured to receive a nozzle.

5. The humidification chamber of claim 4, wherein an inner diameter of the nozzle is configured to decrease along a length of the nozzle so as to increase a velocity of the gases flow prior to the gases flow being introduced to the humidification chamber.

6. The humidification chamber of any one of the preceding claims, wherein the gases inlet comprises a substantially tubular body.

7. The humidification chamber of any one of the preceding claims, wherein an inner diameter of the gases outlet is greater than an inner diameter of the gases inlet.

8. The humidification chamber of claim 7, wherein
   the ratio of the inner diameters of the gases outlet to the inner diameter of the gases inlet is between 3:1 and 7:1, and is preferably approximately 5:1 and/or the ratio of an inner diameter of the gases inlet to a diameter of a bottom wall of the humidification chamber is between 1:25 and 1:10, and is preferably between 1:20 and 1:15.

9. The humidification chamber of any one of the preceding claims, wherein:

    an inner surface of the top and/or sidewall of the humidification chamber, and/or the inlet, is configured to produce a turbulent gases flow within the humidification chamber, and/or
    the inner surface of the top and/or sidewall of the humidification chamber, and/or the inlet, comprises at least one protrusion projecting into the chamber and/or at least one recess recessed away from the chamber to produce turbulent gases flow in the humidification chamber, and/or
    the inlet extends through a side wall of the humidification chamber, and/or
    the inlet and/or the side wall is configured such that gases entering the humidification chamber via the gases inlet swirl into a spiral within the humidification chamber before exiting the humidification chamber through the gases outlet, and/or
    the inlet and/or the side wall is configured such that gases entering the humidification chamber via the gases inlet swirl into a spiral within the humidification chamber before exiting the humidification chamber through the gases outlet and the inlet is disposed on and/or orientated relative to the side wall such that a distance between adjacent winds and/or turns of the spiral is reduced when a flow rate of the gases entering the humidification chamber is increased; and/or the gases are introduced such that the bulk or average gases flow attached to the side wall and follows the shape of the side wall.

10. The humidification chamber of any one of the preceding claims, wherein the flow path through the gases inlet is angled down towards the base of the humidification chamber.

11. The humidification chamber of any one of the preceding claims, further comprising at least one internal element disposed between the gases inlet and the gases outlet to direct or redirect or influence the gases flow within the humidification chamber.

12. The humidification chamber of claim 11, wherein:

the at least one internal element is a baffle shaped and configured to encourage rotation of the bulk gases flow, and/or
at least one said internal element is orientated in a plane substantially parallel to the base or the side wall of the humidification chamber.

13. A humidification chamber of any one of the preceding claims wherein the gases inlet has a longitudinal axis which is substantially parallel to the tangent to the side wall and is located adjacent the side wall;
wherein the gases inlet is configured to introduce the gases flow to the humidification chamber adjacent the side wall at a velocity sufficient to causes the gases to attach to the side wall.

14. The humidification chamber of any one of the preceding claims, wherein the top of the humidification chamber comprises a dome shape.

15. A humidification apparatus for use in a medical humidification system, the humidification apparatus comprising at least two humidification chambers, each of the chambers comprising a humidification chamber according to any one of the preceding claims.

**Patentansprüche**

1. Befeuchtungskammer zur Verwendung in einem medizinischen Befeuchtungssystem, wobei die Befeuchtungskammer umfasst:

einen Boden und eine Oberseite, die durch eine Seitenwand verbunden sind, um die Kammer zu definieren;
einen Gaseinlass, der dazu ausgelegt ist, einen Gasstrom von einer Gasquelle zu empfangen; und
einen Gasauslass,
wobei der Gaseinlass dazu ausgelegt ist, den Gasstrom als einen Gasstrahl in eine Richtung im Wesentlichen tangential zu der Seitenwand der Befeuchtungskammer in die Befeuchtungskammer einzuführen, und **dadurch gekennzeichnet, dass** der Gaseinlass eine Düse umfasst, die eine Verengung hat, die sich nach innen verjüngt, um entlang ihrer Länge schrittweise enger zu werden, wobei das Innenprofil im Wesentlichen glatt ist.

2. Befeuchtungskammer nach Anspruch 1, wobei die Seitenwand eine im Wesentlichen kreisförmige Kammer definiert, wenn von oben betrachtet.

3. Befeuchtungskammer nach Anspruch 1 oder 2, wobei der Gasauslass oben an der Befeuchtungskammer angeordnet ist.

4. Befeuchtungskammer nach einem der vorhergehenden Ansprüche, wobei der Gaseinlass eine Düse umfasst oder dazu ausgelegt ist, eine Düse aufzunehmen.

5. Befeuchtungskammer nach Anspruch 4, wobei ein Innendurchmesser der Düse dazu ausgelegt ist, entlang einer Länge der Düse kleiner zu werden, um eine Geschwindigkeit des Gasstroms zu erhöhen, bevor der Gasstrom in die Befeuchtungskammer eingeführt wird.

6. Befeuchtungskammer nach einem der vorhergehenden Ansprüche, wobei der Gaseinlass einen im Wesentlichen röhrenförmigen Körper umfasst.

7. Befeuchtungskammer nach einem der vorhergehenden Ansprüche, wobei ein Innendurchmesser des Gasauslasses größer ist als ein Innendurchmesser des Gaseinlasses.

8. Befeuchtungskammer nach Anspruch 7, wobei

das Verhältnis des Innendurchmessers des Gasauslasses zu dem Innendurchmesser des Gaseinlasses zwischen 3:1 und 7:1 und vorzugsweise bei ungefähr 5:1 liegt, und/oder
das Verhältnis eines Innendurchmessers des Gaseinlasses zu einem Durchmesser einer Bodenwand der Befeuchtungskammer zwischen 1:25 und 1:10 und vorzugsweise zwischen 1:20 und 1:15 liegt.

9. Befeuchtungskammer nach einem der vorhergehenden Ansprüche, wobei:

eine Innenfläche der Oberseite und/oder Seitenwand der Befeuchtungskammer und/oder des Einlasses dazu ausgelegt ist, einen turbulenten Gasstrom in der Befeuchtungskammer zu erzeugen, und/oder
die Innenfläche der Oberseite und/oder Seitenwand der Befeuchtungskammer und/oder der Einlass mindestens einen Vorsprung umfasst, der in die Kammer und/oder mindestens eine Aussparung, die aus der Kammer ausgespart ist, vorspringt, um einen turbulenten Gasstrom in der Befeuchtungskammer zu erzeugen, und/oder
sich der Einlass durch eine Seitenwand der Befeuchtungskammer erstreckt und/oder
der Einlass und/oder die Seitenwand derart ausgelegt ist, dass über den Gaseinlass in die Befeuchtungskammer eintretende Gase in der Befeuchtungskammer zu einer Spirale verwirbeln, bevor sie die Befeuchtungskammer durch den Gasauslass verlassen, und/oder
der Einlass und/oder die Seitenwand derart ausgelegt sind, dass sich über den Gaseinlass in die Befeuchtungskammer eintretende Gase in der Befeuchtungskammer zu einer Spirale verwirbeln, bevor sie die Befeuchtungskammer durch den Gasauslass verlassen und der Einlass derart an der Seitenwand angeordnet und/oder bezogen darauf ausgerichtet ist, dass ein Abstand zwischen benachbarten Windungen und/oder Drehungen der Spirale reduziert wird, wenn eine Strömungsgeschwindigkeit der in die Befeuchtungskammer eintretenden Gase erhöht wird; und/oder die Gase derart eingeführt werden, dass der Bulk- oder der durchschnittliche Gasstrom an der Seitenwand andockt und der Form der Seitenwand folgt.

10. Befeuchtungskammer nach einem der vorhergehenden Ansprüche, wobei der Strömungsweg durch den Gaseinlass in Richtung des Bodens der Befeuchtungskammer abgewinkelt ist.

11. Befeuchtungskammer nach einem der vorhergehenden Ansprüche, ferner umfassend mindestens ein Innenelement, das zwischen dem Gaseinlass und dem Gasauslass angeordnet ist, um den Gasstrom innerhalb der Befeuchtungskammer zu lenken, umzulenken oder zu beeinflussen.

12. Befeuchtungskammer nach Anspruch 11, wobei:

das mindestens eine Innenelement eine Ablenkplatte ist, die dazu geformt und ausgelegt ist, die Rotation des Bulkgasstroms zu fördern und/oder
mindestens ein derartiges Innenelement in einer Ebene im Wesentlichen parallel zum Boden oder zur Seitenwand der Befeuchtungskammer ausgerichtet ist.

13. Befeuchtungskammer nach einem der vorhergehenden Ansprüche, wobei der Gaseinlass eine Längsachse hat, die im Wesentlichen parallel zur Tangente der Seitenwand verläuft und sich neben der Seitenwand befindet; wobei der Gaseinlass dazu ausgelegt ist, den Gasstrom neben der Seitenwand mit einer Geschwindigkeit in die Befeuchtungskammer einzuführen, die ausreicht, um zu bewirken, dass die Gase an der Seitenwand andocken.

14. Befeuchtungskammer nach einem der vorhergehenden Ansprüche, wobei die obere Seite der Befeuchtungskammer eine Kuppelform umfasst.

15. Befeuchtungsvorrichtung zur Verwendung in einem medizinischen Befeuchtungssystem, wobei die Befeuchtungsvorrichtung mindestens zwei Befeuchtungskammern umfasst, wobei jede der Kammern eine Befeuchtungskammer nach einem der vorhergehenden Ansprüche umfasst.

**Revendications**

1. Chambre d'humidification destinée à être utilisée dans un système d'humidification médical, la chambre d'humidification comprenant :

   une base et une partie supérieure reliées par une paroi latérale pour définir la chambre ;
   une entrée de gaz configurée pour recevoir un écoulement de gaz provenant d'une source de gaz ; et
   une sortie de gaz,
   l'entrée de gaz étant orientée par rapport à la paroi latérale de la chambre pour introduire l'écoulement de gaz dans la chambre d'humidification sous la forme d'un jet de gaz dans une direction sensiblement tangentielle à la paroi latérale de la chambre d'humidification et **caractérisée en ce que** l'entrée de gaz comprend une buse ayant une constriction qui s'amincit intérieurement de manière à se rétrécir progressivement sur sa longueur, le profil interne étant sensiblement lisse.

2. Chambre d'humidification selon la revendication 1, la paroi latérale définissant une chambre sensiblement circulaire, en vue de dessus.

3. Chambre d'humidification selon la revendication 1 ou 2, la sortie de gaz étant disposée sur le dessus de la chambre d'humidification.

4. Chambre d'humidification selon l'une quelconque des revendications précédentes, l'entrée de gaz comprenant ou étant configurée pour recevoir une buse.

5. Chambre d'humidification selon la revendication 4, un diamètre intérieur de la buse étant configuré pour diminuer sur une longueur de la buse de manière à augmenter la vitesse de l'écoulement de gaz avant que l'écoulement de gaz ne soit introduit dans la chambre d'humidification.

6. Chambre d'humidification selon l'une quelconque des revendications précédentes, l'entrée de gaz comprenant un corps sensiblement tubulaire.

7. Chambre d'humidification selon l'une quelconque des revendications précédentes, le diamètre intérieur de la sortie de gaz étant supérieur au diamètre intérieur de l'entrée de gaz.

8. Chambre d'humidification selon la revendication 7,
   le rapport entre les diamètres intérieurs de la sortie de gaz et le diamètre intérieur de l'entrée de gaz étant compris entre 3:1 et 7:1, et étant de préférence d'environ 5:1 et/ou le rapport entre un diamètre intérieur de l'entrée de gaz et un diamètre d'une paroi de fond de la chambre d'humidification étant compris entre 1:25 et 1:10, et étant de préférence compris entre 1:20 et 1:15.

9. Chambre d'humidification selon l'une quelconque des revendications précédentes,

   une surface intérieure de la partie supérieure et/ou de la paroi latérale de la chambre d'humidification, et/ou de l'entrée, étant configurée pour produire un écoulement de gaz turbulent à l'intérieur de la chambre d'humidification, et/ou
   la surface intérieure de la partie supérieure et/ou de la paroi latérale de la chambre d'humidification, et/ou l'entrée, comprenant au moins une protubérance faisant saillie dans la chambre et/ou au moins un renfoncement en retrait de la chambre pour produire un écoulement de gaz turbulent dans la chambre d'humidification, et/ou l'entrée s'étendant à travers une paroi latérale de la chambre d'humidification, et/ou
   l'entrée et/ou la paroi latérale étant configurées de telle sorte que les gaz entrant dans la chambre d'humidification par l'entrée de gaz tourbillonnent en spirale à l'intérieur de la chambre d'humidification avant de sortir de la chambre d'humidification par la sortie de gaz, et/ou
   l'entrée et/ou la paroi latérale étant configurées de telle sorte que les gaz entrant dans la chambre d'humidification par l'entrée de gaz tourbillonnent en spirale à l'intérieur de la chambre d'humidification avant de sortir de la chambre d'humidification par la sortie de gaz, et l'entrée étant disposée sur et/ou orientée par rapport à la paroi latérale de telle sorte qu'une distance entre les vents adjacents et/ou les tours de la spirale est réduite lorsqu'un débit des gaz entrant dans la chambre d'humidification est augmenté ; et/ou les gaz étant introduits de telle sorte que les gaz en vrac ou moyens s'écoulent de en étant fixés à la paroi latérale et suivent la forme de la paroi latérale.

**10.** Chambre d'humidification selon l'une quelconque des revendications précédentes, le chemin d'écoulement à travers l'entrée de gaz étant incliné vers le bas en direction de la base de la chambre d'humidification.

**11.** Chambre d'humidification selon l'une quelconque des revendications précédentes, comprenant en outre au moins un élément interne disposé entre l'entrée de gaz et la sortie de gaz pour diriger, rediriger ou influencer l'écoulement de gaz à l'intérieur de la chambre d'humidification.

**12.** Chambre d'humidification selon la revendication 11,

l'au moins un élément interne étant une déflecteur formé et configuré pour encourager la rotation de l'écoulement de gaz en vrac, et/ou
au moins un de ces éléments internes étant orienté dans un plan sensiblement parallèle à la base ou à la paroi latérale de la chambre d'humidification.

**13.** Chambre d'humidification selon l'une quelconque des revendications précédentes, l'entrée de gaz ayant un axe longitudinal sensiblement parallèle à la tangente à la paroi latérale et étant située à côté de la paroi latérale ; l'entrée de gaz étant configurée pour introduire l'écoulement de gaz dans la chambre d'humidification adjacente à la paroi latérale à une vitesse suffisante pour que les gaz se fixent à la paroi latérale.

**14.** Chambre d'humidification selon l'une quelconque des revendications précédentes, la partie supérieure de la chambre d'humidification ayant la forme d'un dôme.

**15.** Appareil d'humidification destiné à être utilisé dans un système d'humidification médical, l'appareil d'humidification comprenant au moins deux chambres d'humidification, chacune des chambres comprenant une chambre d'humidification selon l'une quelconque des revendications précédentes.

FIG. 1

EP 3 773 849 B1

**FIG. 2**

**FIG. 2A**

FIG. 2B

FIG. 2C

FIG. 2D

FIG. 2E

FIG. 3A

FIG. 3B

FIG. 4

FIG. 5

FIG. 6

FIG. 7

FIG. 8

FIG. 9

FIG. 10A

FIG. 10B

**FIG. 11**

**FIG. 12**

**FIG. 13**

FIG. 14A

FIG. 14B

FIG. 15A

FIG. 15B

FIG. 16

FIG. 17

FIG. 18

FIG. 19

**FIG. 20A**

**FIG. 20B**

**FIG. 21**

**FIG. 22**

2218a

2216a

2214a

2225a

2222a

## FIG. 22A

2318

2316

2326

2325

2314

2322

Heater Plate

## FIG. 23

2418

2416

2414

2425

2422   Heater Plate Fins

## FIG. 24

FIG. 25

FIG. 26A

FIG. 26B

FIG. 27

FIG. 28

FIG. 29

FIG. 30

3116 ↗

3018    3131

Heater plate

3114    3125

3116a

3116b

Air Gap
Insulation

**FIG. 31**

Suction

3232    3216 ↙

Inlet

**FIG. 32**

3233

3316    3314

**FIG. 33**

3413

3416

3414

## FIG. 34

3535

3516

3514

## FIG. 35

**Vortex Chamber Efficiency Comparision**

FIG. 36

EP 3 773 849 B1

3716

FIG. 37

FIG. 38

FIG. 39

FIG. 40

FIG. 41

4218

4216

4214

FIG. 42

4216

4218

4214

FIG. 43

FIG. 44

FIG. 45

4614

4618

4614a

4616

4614b

FIG. 46

4614

4616

4618

4614a

4614b

FIG. 47

FIG. 48

FIG. 49

FIG. 50

**MR810 Humidity Output with MR225
and Vortex chambers**

FIG. 51

**MR810 Humidity Output with MR225
and Vortex chambers**

FIG. 52

**MR810 Humidity Output with MR225
and Vortex chambers**

FIG. 53

**MR810 Humidity Output with MR225
and Vortex chambers**

FIG. 54

FIG. 55

FIG. 56

FIG. 57

FIG. 58

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017035984 A1
- US 4943704 A
- US 2017197057 A1
- EP 2842589 A2
- US 2016184547 A
- WO 2017126982 A1
- WO 2007101298 A1
- JP 2004057278 A
- US 20170035984 A1
- WO 2018106127 A
- WO 2018097738 A
- WO 2011056080 A
- WO 2015038013 A